# FASCICULE DE BREVET EUROPEEN

(11) **EP 0 876 495 B1**
(45) Date de publication et mention de la délivrance du brevet: **30.11.2005**
(21) Numéro de dépôt: 96931123.2
(22) Date de dépôt: 13.09.1996
(51) Int. Cl.: C12N 15/81, C12N 9/02, C12N 1/19, C12Q 1/26

(54) **SOUCHES DE LEVURES GENETIQUEMENT MODIFIEES**
GENTECHNISCH HERGESTELLTE HEFESTÄMME
GENETICALLY ENGINEERED YEAST STRAINS

(30) Priorité: 15.09.1995 FR 9510826
(43) Date de publication de la demande: 11.11.1998
(73) Titulaire: Aventis Pharma S.A., 92160 Antony (FR); CENTRE NATIONAL DE LA RECHERCHE SCIENTIFIQUE (CNRS), 75016 Paris (FR)
(72) Inventeur: BELLAMINE, Aouatef, F-75013 Paris (FR); DELORME, Frédéric, F-91120 Palaiseau (FR); PERRET, Alain, F-78450 Villepreux (FR); POMPON, Denis, F-91190 Gif-sur-Yvette (FR)
(74) Mandataire: Savina, Jacques
(86) Numéro de dépôt international: PCT/FR1996/001413
(87) Numéro de publication internationale: WO 1997/010344

(56) Documents cités:
- CHEMICAL ABSTRACTS, vol. 117, no. 9, 31 Août 1992 Columbus, Ohio, US; abstract no. 85812, PEYRONNEAU, MARIE ANNE ET AL: "Optimization of yeast -expressed human liver cytochrome P450 3A4 catalytic activities by coexpressing NADPH - cytochrome P450 reductase and cytochrome b5" XP002005489 & EUR. J. BIOCHEM. (1992), 207(1), 109-16 CODEN: EJBCAI;ISSN: 0014-2956,
- CHEMICAL ABSTRACTS, vol. 115, no. 1, 8 Juillet 1991 Columbus, Ohio, US; abstract no. 147, GUENGERICH, F. PETER ET AL: "Oxidation of dihydropyridine calcium channel blockers and analogs by human liver cytochrome P-450 IIIA4" XP002005490 & J. MED. CHEM. (1991), 34(6), 1838-44 CODEN: JMCMAR;ISSN: 0022-2623,
- CHEMICAL ABSTRACTS, vol. 114, no. 11, 18 Mars 1991 Columbus, Ohio, US; abstract no. 96211, RENAUD, JEAN PAUL ET AL: "Expression of human liver cytochrome P450 IIIA4 in yeast. A functional model for the hepatic enzyme" XP002005491 & EUR. J. BIOCHEM. (1990), 194(3), 889-96 CODEN: EJBCAI;ISSN: 0014-2956,
- GENE (1993), 125(1), 49-55 CODEN: GENED6;ISSN: 0378-1119, XP002005488 TRUAN, GILLES ET AL: "Enhanced in vivo monooxygenase activities of mammalian P450s in engineered yeast cells producing high levels of NADPH -P450 reductase and human cytochrome b5" cité dans la demande
- CHEMICAL ABSTRACTS, vol. 112, no. 19, 7 Mai 1990 Columbus, Ohio, US; abstract no. 173291, YAMANO, SHIGERU ET AL: "Human NADPH-P450 oxidoreductase: complementary DNA cloning, sequence and vaccinia virus-mediated expression and localization of the CYPOR gene to chromosome 7" XP002005492 & MOL. PHARMACOL. (1989), 36(1), 83-8 CODEN: MOPMA3;ISSN: 0026-895X, 1989, cité dans la demande
- CHEMICAL ABSTRACTS, vol. 112, no. 3, 15 Janvier 1990 Columbus, Ohio, US; abstract no. 17111, MIYATA, MASAAKI ET AL: "Isolation and characterization of human liver cytochrome b5 cDNA" XP002005493 & PHARMACOL. RES. (1989), 21(5), 513-20 CODEN: PHMREP, 1989, cité dans la demande
- CHEMICAL ABSTRACTS, vol. 119, no. 19, 8 Novembre 1993 Columbus, Ohio, US; abstract no. 199224e, URBAN,PHILIPPE ET AL.: "Xenobiotic metabolism in humanized yeast:Engineered yeast cells producing human NADPH-cytochrome P-450 reductase,cytochrome b5,epoxide hydrolase and P-450s." page 464; XP002005494 & BIOCHEM.SOC.TRANS., vol. 21, no. 4, 1993, pages 1028-1034, cité dans la demande

## Description

La présente invention concerne de nouvelles souches de levures exprimant une activité cytochrome P450 et leur utilisation. Elle se rapporte en particulier à des souches de levures capables de produire un système d'enzymes cytochromes P450 humaines et les plasmides utilisés pour leur construction.

Les cytochromes P450 constituent une super famille d'enzymes membranaires. Ce sont des monooxygénases qui interviennent plus particulièrement dans le métabolisme des xénobiotiques et des médicaments.

Elles sont notamment utilisées dans :
- le diagnostic in vitro de la formation de métabolites toxiques ou mutagènes par le métabolisme hépatique humain de molécules xénobiotiques naturelles ou artificielles (polluants médicaments ou additifs). Ce diagnostic est primordial pour le développement de nouvelles molécules pharmaceutiques,
- l'identification et la destruction de molécules toxiques ou polluantes de l'environnement et
- la production de métabolites.

Du fait de leur implication, à la fois dans ces processus de détoxication et ces phénomènes de toxicité, ces protéines ont été largement étudiées (Guenguerich, 1988).

Toutefois, ces études se sont heurtées rapidement à des difficultés telle que l'étude de formes individuelles de cytochromes P450. Pour pallier à ces problèmes, les systèmes d'expression hétérologues ont alors été développés.

L'utilisation des cellules mammifères comme hôtes d'expression hétérologue a été développée depuis 1986 (Zuber et al., 1986). Ces systèmes ont l'avantage d'être proches des cellules hépatiques (localisation majoritaire des cytochromes P450), mais souffrent malheureusement de bas niveaux d'expression.

En ce qui concerne les hôtes procaryotes, tels que les bactéries, ils permettent certes d'avoir des quantités importantes de cytochrome P450 correctement repliés (Barnes et al., 1991), mais avec ce type d'hôtes on observe des modifications incontournables de la partie 5'terminale exprimée de l'ADN (Doehmer & Greim, 1992).

En revanche le choix d'hôtes eucaryotes de type levure est tout particulièrement avantageux : cet organisme permet de se mettre dans des conditions proches de celles des cellules hépatiques humaines et conduit à un niveau d'expression en protéines élevé. En outre la levure possède sous forme endogène toute la machinerie enzymatique nécessaire à l'expression des protéines membranaires, du type cytochrome P450, et de leurs enzymes associés, c'est ainsi qu'elle dispose d'un cytochrome b5 et d'une NADPH-cytochrome P450 réductase, deux enzymes dont la présence est nécessaire au fonctionnement du cytochrome P450.

La levure offre donc une solution avantageuse aux différents problèmes (Oeda K. et al., 1985; Pompon, 1988), puisque avec cet organisme :
- les protéines exprimées n'ont pas besoin d'être modifiées au niveau de leur séquence N-terminale (comme pour l'expression dans la bactérie)
- on obtient des quantités raisonnables de cytochrome P450 hétérologues pour différentes études biochimiques et structurales,
- un système d'enzymes associés y existe déjà.

Parmi les levures particulièrement étudiées pour l'expression de protéines hétérologues, on peut citer notamment *Kluyveromyces, Pichia, Hansenula, Candida* et *Saccharomyces* dont on connaît bien la structure du génome. Différents systèmes d'expression de cytochrome P450 dans des levures ont été décrits dans la littérature.

Dans les souches dites de première génération les cytochromes P450 ont été exprimés à partir de plasmides et utilisent comme donneurs d'électrons la NADPH-cytochrome P450 réductase et le cytochrome b₅ endogènes de levure (Pompon, 1988; Cullin & Pompon, 1988).

Une première amélioration de ce système a donné lieu à des souches dites de deuxième génération où la cytochrome P450 réductase de la levure a été surexprimée (sous le contrôle du promoteur GAL10-CYC1) et un cytochrome b₅ humain coexprimé (brevet WO93/02200et Truan et al., 1993). Ces souches ont ainsi permis d'obtenir des activités enzymatiques du cytochrome P450 recombinant 5 à 60 fois plus importantes selon l'isoforme que dans la souche de départ.

Toutefois les systèmes existants ne donnent pas entièrement satisfaction : soit ils ne permettent pas d'avoir une expression suffisante des protéines, soit les protéines obtenues ne sont pas assez proches du système humain.
La présente invention à précisément pour objectif de proposer une troisième génération de souche ne présentant pas les inconvénients précités. De manière inattendue, la demanderesse a mis en évidence qu'il était possible de remplacer simultanément la NADPH cytochrome P450 réductase et le cytochrome b5 de levure par leurs homologues humains. Ceci est d'autant plus surprenant que la disruption simultanée de ces deux gènes était connue pour être létale chez la levure et que jusqu'à présent il n'était pas possible d'obtenir une souche viable ayant les deux gènes délétés.

Dans les souches revendiquées, la cytochrome P450 réductase et/ou le cytochrome b5 de levure ont été substitués par leur homologue humain. Ceci permet très avantageusement la création d'un système très proche des cellules hépatiques, étant donné que tout le système multienzymatique est alors de même nature.
Ce nouveau système, permet d'étudier l'effet de la nature des partenaires rédox des cytochromes P450 exprimés ainsi que les stoechiométries nécessaires à des activités cytochrome P450 comparables à celles qui existent dans le foie.

Le premier objet de l'invention réside donc dans une souche de levure génétiquement modifiée caractérisée en ce que:
1° Les gènes codant pour le cytochrome b5 endogène et pour la NADPH-cytochrome P450- réductase endogène ont été inactives,
2° Elle contient un acide nucléique codant pour la NADPH-cytochrome P450-réductase humaine,
3° Elle contient un acide nucléique codant pour le cytochrome b5 humain.

Les acides nucléiques utilisés pour intégrer dans la souche les gènes codant pour le cytochrome b5 humain et la réductase humaine sont préférentiellement des ADNc. Les ADNc contenant la totalité de la séquence codante pour ces deux protéines ont été isolés et séquencés (pour la réductase humaine voir S Yamano et al. Mol. Pharmacol. 1989 vol. 36 : 83-8, et pour le cytochrome b5 humain voir M Miyata et al. Pharmacol. Res. 1989 vol. 21 : 513-20).

Tout aussi préférentiellement la levure choisie est *Saccharomyces cerevisiae*

Au sens de la présente invention, on entend par gènes inactivés, un gène rendu incapable de coder pour sa protéine naturelle. L'incapacité desdits gènes'à coder pour leurs protéines naturelles peut se manifester soit par la production d'une protéine inactive en raison de modifications structurales ou conformationnelles, soit par l'absence de production, soit par la production de la protéine naturelle à un niveau atténué.

L'inactivation des gènes natifs peut être obtenue selon différentes méthodes:
- une délétion totale ou partielle du gène. Par délétion on entend toute suppression du gène considéré. Il peut s'agir d'une partie de la région codant pour la protéine et/ou de tout ou partie de la région promotrice de la transcription.
- une ou plusieurs mutations ponctuelles dans le gène. Les mutations peuvent être obtenues par un traitement à l'aide d'agents mutagènes, chimiques (comme les agents alkylants, bialkylants ou intercalants) ou d'agents mutagènes physiques (rayons X.g. ultraviolet), ou par mutagenèse dirigée.
- une insertion mutationelle par action d'enzymes de restriction qui vont interrompre le cadre de lecture du gène et l'inactiver et/ou,
- une disruption génique par exemple selon le protocole initialement décrit par Rothstein [Meth. Enzymol. (1983)202]. Dans ce cas, l'intégralité de la séquence codante sera perturbée pour permettre le remplacement par recombinaison homologue de la séquence sauvage par une séquence codant pour la protéine humaine correspondante.

Selon la présente invention, on utilise préférentiellement la méthode de disruption génique comme décrit ci-aprés.

Pour la transformation des souches revendiquées en vu de leur faire exprimer des enzymes humains selon l'invention, différentes solutions sont envisageables ; d'une part on peut transformer une souche sauvage, dont l'un des gènes a été inactivé, par un plasmide réplicatif contenant l'acide nucléique codant pour la protéine humaine correspondante. Dans ce cas l'acide nucléique n'est pas intégré dans le génome de la levure.

D'autre part on peut intégrer un acide nucléique, sous la forme d'un ADNc comprenant la séquence codant pour la protéine humaine concernée, dans le génome de la levure. Dans ce cas, l'intégration peut se faire soit dans un locus connu sur ce génome correspondant à un gène marqueur, n'altérant ni les propriétés de reproduction de la levure ni la viabilité de celle-ci, soit à la place occupée par le gène natif inactivé.

L'acide nucléique codant pour la réductase de même que l'acide nucléique codant pour le cytochrome b5, peuvent donc être introduits dans la souche selon l'une de ces méthodes.

Selon la présente invention, pour améliorer la stabilité de la souche et se placer dans des conditions plus favorables, on choisit de préférence le mode de réalisation consistant à intégrer l'acide nucléique codant pour la NADPH-cytochrome P450-réductase humaine et/ou l'acide nucléique codant pour le cytochrome b5 humain dans le génome de la levure, un mode préférentiel de réalisation de la présente invention étant d'intégrer ces acides nucléiques en lieu et place des gènes endogènes.

Selon un autre mode préféré de réalisation de la présente invention on intègre le gène codant pour le cytochrome b5 humain dans un site intergénique pour un gène marqueur en particulier dans le site intergénique SPL1/leu2.

Une autre caractéristique de l'invention réside dans une souche caractérisée en ce que l'acide nucléique codant pour le cytochrome b5 humain est intégré dans le génome.

Un mode de réalisation particulier de l'invention consiste à faire figurer deux copie du cytochrome b5 dans la souche transformée.

Un autre problème rencontré par la demanderesse est celui de l'expression des gènes humains dans la levure a un taux suffisant.
Il est avantageux pour cela que ces gènes soit mis sous le contrôle d'un promoteur de levure qui permet que ceux-ci soient exprimés. Ces promoteurs de levure peuvent, soit être inductibles, soit être constitutifs. Dans la présente demande on entends par promoteur constitutif un promoteur dont l'expression est constante dans les conditions standard de culture. Selon la présente invention l'un au moins des gènes humains est sous le contrôle d'un promoteur constitutif de levure.
Ce promoteur est choisi parmi les promoteurs connus. On peut par exemple utiliser les promoteurs des gènes de l'isocytochrome C1 (CYC1), de l'alcool déshydrogénase (ADH1), du facteur d'élongation de la transcription (TEF), de la glyceraldéhyde phosphodéhydrogénase de levure (ci-après GAPDH) et de la phosphoglyceratekinase de levure (ci-après PGK).
Préférentiellement le promoteur est choisi parmi le promoteur du gène de la glyceraldéhyde phosphodéhydrogénase de levure, le promoteur du gène de la phosphoglycérate kinase de levure et le promoteur endogéne du cytochrome b5 de levure. Il est à signaler que dans un mode particulier de réalisation de l'invention le gène codant pour le cytochrome b5 humain est sous contrôle du promoteur endogène de Yb5.

En ce qui concerne le promoteur inductible, il est choisi préférentiellement parmi les promoteurs GAL10 et CYC1-GAL10.

Jusqu'à présent on ne pouvait pas obtenir de souches haploïdes présentant les caractéristiques qui nous intéressent c'est à dire les inactivations des gènes endogènes précités et leur remplacement par des acides nucléiques codant pour les gènes humains correspondant. Il fallait rester sous forme diploïde. L'un des avantage de la présente invention est de pouvoir travailler avec des levures haploïdes ce qui permet d'avoir une meilleure stabilité et d'éviter les recombinaisons non désirées.

Selon un mode préférentiel de réalisation de l'invention les souches sont donc caractérisées en ce qu'elles sont haploïdes.

Les souches selon l'invention possèdent au moins un acide nucléique codant pour le cytochrome P450 humain. Le dit acide nucléique sera de préférence intégré sur un plasmide. Les souches de levures humanisées selon l'invention peuvent être transformées selon les techniques habituelles par un plasmide d'expression de cytochrome P450 quelconque, pourvu que le dit plasmide soit compatible au niveau de ses marqueurs de sélection avec les souches de levure développées. En particulier de tels plasmides peuvent être obtenus en clonant selon les règles de l'art la phase codante d'un ADNc codant pour un cytochrome P450 humain quelconque dans le polylinker de clonage du plasmide pYeDP60 (voir matériels et méthodes).
Le cytochrome P450 peut être en particulier choisi parmi les cytochromes P450 humains 1A1, 1A2, 1B1, 2C8, 2C9, 2C18, 2C19, 2E1, 3A4, 3A5. Les souches humanisées selon la présente demande présentent des avantages incontestables, par rapport aux levures sauvages et par rapport aux souches recombinantes préalablement développées, pour l'expression

Un autre objet de l'invention consiste en une souche de levure selon l'invention, dans laquelle on fera s'exprimer, en outre, l'activité monooxygénase d'un cytochrome P450 humain porté par un plasmide.

Un autre mode de réalisation de l'invention consiste à faire figurer la copie supplémentaire de l'acide nucléique codant pour le cytochrome b5 précédement décrite, sur un plasmide et notamment sur celui qui contient déjà une copie de l'acide nucléique codant pour le cytochrome P450. Pour l'obtention d'une activité optimale des P450, il est en effet particulièrement avantageux de pouvoir réaliser une stoechiométrie molaire relative au moins égale à 1/1 entre les niveaux d'expression du cytochrome b5 humain et du P450 humain. L'intégration génomique d'une seule copie du gène du cytochrome b5 peut, dans certaines circonstances, s'avérer insuffisante au regard du niveau élevé d'expression du P450, tel qu'il résulte de son expression à partir d'un plasmide multicopie selon l'invention. La présente invention permet ainsi d'améliorer encore l'efficacité du système en décrivant la construction d'une série de plasmides portant à la fois une cassette d'expression pour le cytochrome P450 d'intérêt et une cassette d'expression pour le cytochrome b5. La structure particulière de ces plasmides permet une expression stable, de haut niveau et de stoechiométrie adéquate entre les deux cytochromes. Ces plasmides sont compatibles avec l'ensemble des souches décrites dans la demande. Leur usage peut également être étendu à d'autres souches.

La présente invention a pour objectif préférentiel de mettre au point une souche de levure répondant à toutes les caractéristiques précédemment décrites. Plusieurs intermédiaires sont décrits pour y parvenir.

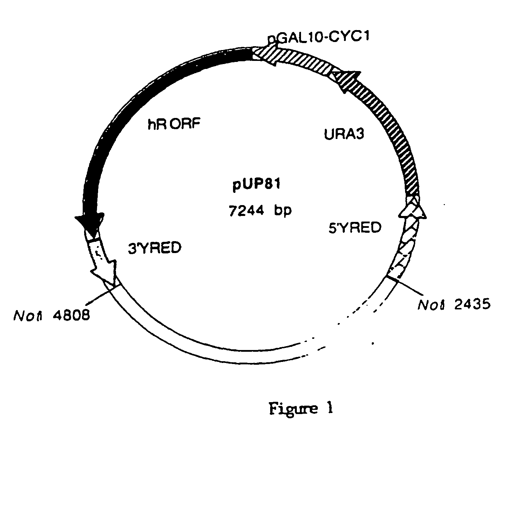

Dans la présente invention on part préférentiellement de souches telles que décrites dans la littérature, et notamment :
- de la souche W(ΔB), décrite par Truant *al,* dont le gène codant pour le cytochrome b5 de levure (ci-après Yb5) a été disrupté. Pour ce faire on construit un vecteur ayant le gène marqueur HIS3 intégré au niveau d'un site de restriction du gène du cytochrome b5. Ce vecteur est utilisé pour transformer une souche HIS3- diploïde. Les recombinants sont sélectionnés,
- de la souche W(R) dont le gène codant pour réductase de levure n'a pas été inactivé.
- et de la souche W(hR), obtenue à partir d'une souche W(RA), par transformation par le vecteur pUP81 (fig 1). Dans cette souche le gène codant pour la réductase de levure (ci-après YRED) inactivé a été remplacé, par insertion d'une cassette contenant le promoteur inductible et la séquence codant pour la réductase humaine (ci-après HRED).

Ces souches sont croisées, puis sporulées et l'on sélectionne les souches W(hR,ΔB) haploïdes qui sont délétées pour Yb5 et YRED et qui expriment la réductase humaine sous contrôle du promoteur GAL10-CYC1.

A cet égard un autre objet de l'invention consiste en une souche comprenant un acide nucléique codant pour la NADPH-cytochrome P450-réductase humaine sous contrôle du promoteur GAL10-CYC1 et dont les gènes codant pour le cytochrome b5 de levure et la NADPH-cytochrome P450-réductase de levure ont été inactivés (souche W(hR, ΔB)).

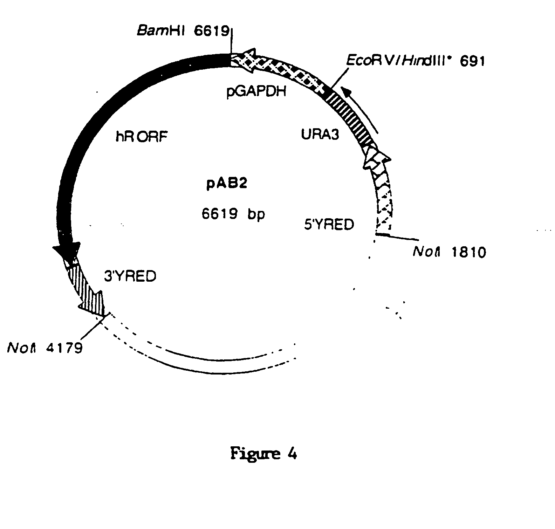

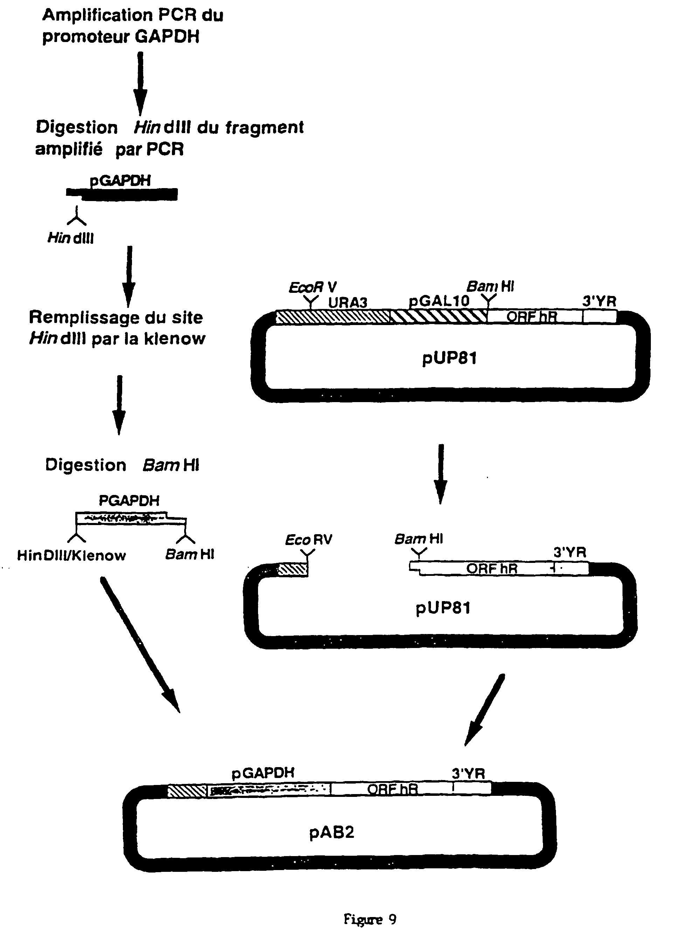

Une autre souche préférée selon l'invention consiste en une souche identique à la précédente dans laquelle le promoteur inductible GAL10-CYC1 a été remplacé par le promoteur GAPDH.
Ce remplacement peut être effectué, par une transformation par le plasmide pAB2 (fig 4), construit à partir de pUP81 (fig 9), qui contient un ADNc codant pour la HRED sous le contrôle du promoteur constitutif de la GAPDH. Les transformants sont sélectionnés selon la méthode décrite dans le brevet WO94/01564. La souche obtenue est désignée par W(GhR,ΔB) Dans cette souche, la séquence codant pour la réductase humaine est sous le contrôle du promoteur constitutif de la GAPDH de levure et le cytochrome b5 et la YRED de levure sont inactivés.
Un autre objet de l'invention consiste en une souche caractérisée en ce que l'acide nucléique codant pour la NADPH-cytochrome P450-réductase humaine est sous contrôle du promoteur du gène de la glyoeraldéhyde phosphodéhydrogénase de levure.

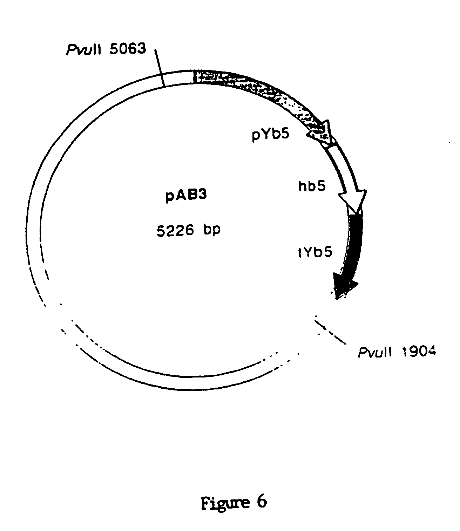

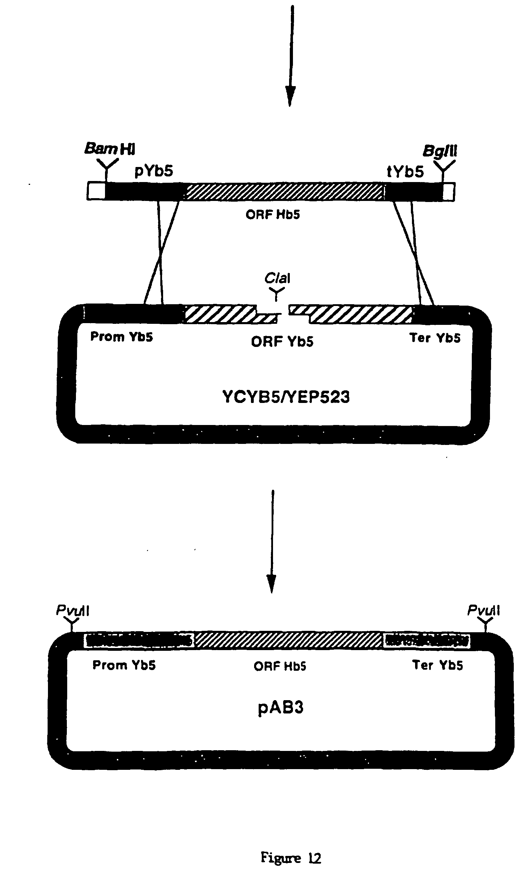

Une autre souche préférée selon l'invention est obtenue à partir de la souche W(hR,ΔB) par transformation par le vecteur pAB3 (fig 6 et 12) contenant la séquence codant pour le cytochrome b5 humain.
On sélectionne les levures ayant intégré celle-ci. La souche ainsi obtenue est nommée W(hR,hb5). Cette souche possède les deux séquences codant pour les protéines humaines.

Pour construire une souche particulièrement avantageuse selon l'invention on transforme la souche W(GhR,ΔB) par le plasmide pAB3, de la même manière que précédemment. On obtient ainsi une souche W(GhR,hb5) qui a les propriétés des deux précédentes à savoir qu'elle exprime le gène codant pour la réductase humaine sous contrôle du promoteur de la GAPDH de levure et qu'elle peut aussi exprimer le gène codant pour le cytochrome b5 humain sous contrôle du promoteur pYb5 de levure.

De manière tout aussi préférentielle la demanderesse a construit une souche à partir de la souche W(hR,ΔB). Cette souche est transformée par le vecteur plasmidique pAP1 (fig 8 et 13) préalablement linéarisé par l'action d'un enzyme de restriction. On sélectionne les transformants qui ont intégré la séquence codant pour le cytochrome b5 humain sous le contrôle du promoteur PGK (phosphoglycérate kinase) de levure, portés par pAP1, au niveau du site intergénique leu2/SPL1 du chromosome de la levure (fig 14).
Cette souche porte dans la nomenclature de la demanderesse le nom W(hR, Lhb5). Elle peut exprimer la séquence codant pour la réductase humaine sous contrôle du promoteur pGAL10-CYC1 et la séquence codant pour le cytochrome b5 sous le contrôle du promoteur PGK de levure.
Un autre objet de l'invention concerne une souche de levure caractérisée en ce qu'elle comprend au moins un acide nucléique codant pour le cytochrome b5 humain sous contrôle du promoteur du gène de la phosphoglycérate kinase de levure.

Tout particulièrement on préfère la souche suivante. On part soit de la souche W(GhR,ΔB) que l'on transforme par le vecteur pAP1 linéarisé. On sélectionne les clones qui ont intégré au site chromosomique précité leu2/SPL1 la séquence codant pour le cytochrome b5 humain sous le contrôle du promoteur PGK de levure.
On obtient alors une souche W(GhR, Lhb5) contenant les séquences codantes pour les deux gènes humains sous contrôle de deux promoteurs constitutifs de levure.
Un autre objet de l'invention est caractérisé en ce que, dans une souche, on a à la fois :
- L'acide nucléique codant pour la NADPH-cytochrome P450-réductase humaine sous contrôle du promoteur du gène de la glyceraldéhyde phosphodéhydrogénase de levure.
- Et l'acide nucléique codant pour le cytochrome b5 humain sous contrôle du promoteur du gène de la phosphoglycérate kinase de levure.

Un autre mode de réalisation de l'invention consiste à partir de la souche W(R) et de la transformer par le vecteur pAP1, après sélection on obtient une souche W(R, Lhb5,Yb5)

Préférentielement on part de la souche W(hR) que l'on transformera de la même manière, après sélection on obtient une souche W(hR, Lhb5, Yb5)

Les souches construites selon la présente invention permettent la mise en oeuvre de procédés visant à l'évaluation de la toxicité des métabolites provenant de la dégradation par le système enzymatique cytochrome P450 de nouvelles molécules chimiques.

Un autre objet de l'invention concerne un procédé d'évaluation de la toxicité d'un composé caractérisé en ce que :
- Le dit composé est mis en présence d'une levure selon l'invention ou d'une préparation enzymatique dérivée d'une telle levure, et on analyse les métabolites produits quant à leur toxicité.

La présente invention permet de plus d'avoir un complexe enzymatique très proche de celui existant dans les cellules hépatiques humaines. Ceci offre la possibilité de travailler in vitro dans de bonnes conditions d'expression des enzymes humains. Ainsi on peut déterminer quels seront les métabolites résultant, chez l'homme, de la dégradation par le complexe cytochrome P450, de nouveau composés chimiques.
Un autre objet de l'invention concerne une méthode de détermination in vitro des métabolites humain d'un composé chimique caractérisée en ce que :
- Le dit composé est mis en présence d'une levure selon l'invention ou d'une préparation enzymatique dérivée d'une telle levure, et on identifie les métabolites produits.

La présente invention est décrite plus en détail à l'aide des exemples qui suivent, qui doivent être considérés comme illustratifs et non limitatifs.

### DESCRIPTIONS DES FIGURES

Figure 1: plasmide pUP81.

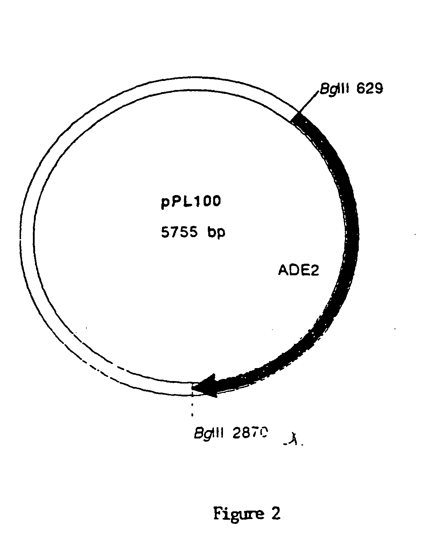

Figure 2: plasmide pPL100.

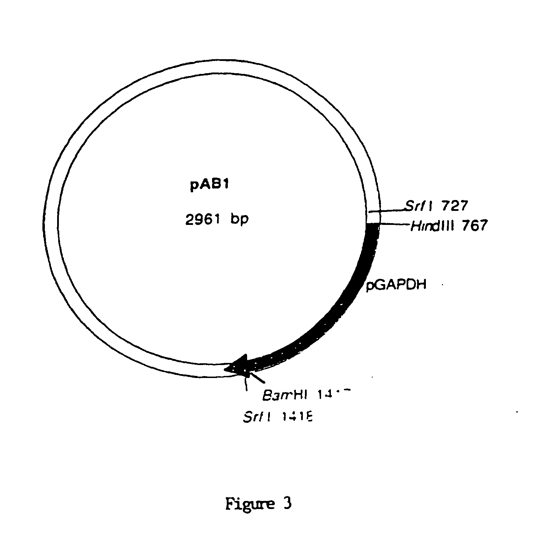

Figure 3: plasmide pAB1.
Figure 4: plasmide pAB2.

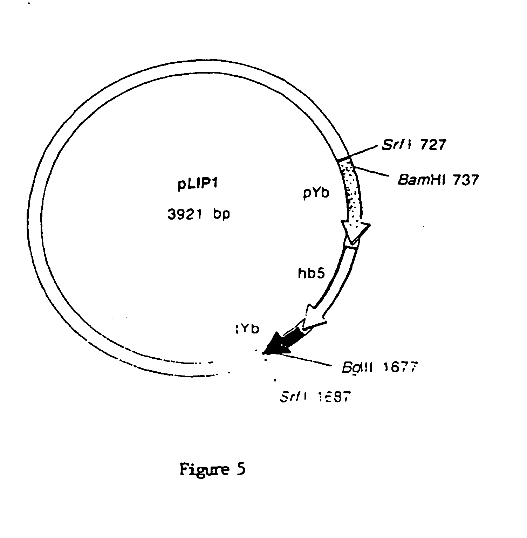

Figure 5: plasmide pLIP1.
Figure 6: plasmide pAB3.

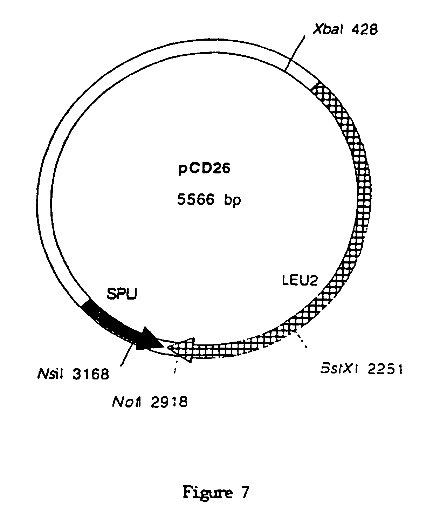

Figure 7: plasmide pCD26.

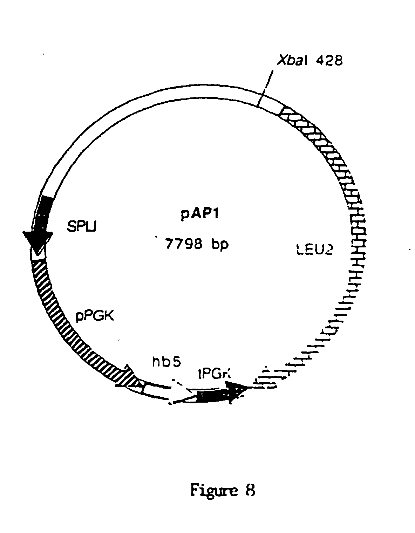

Figure 8: plasmide pAP1.
Figure 9: construction de pAB2 à partir de pUP81.

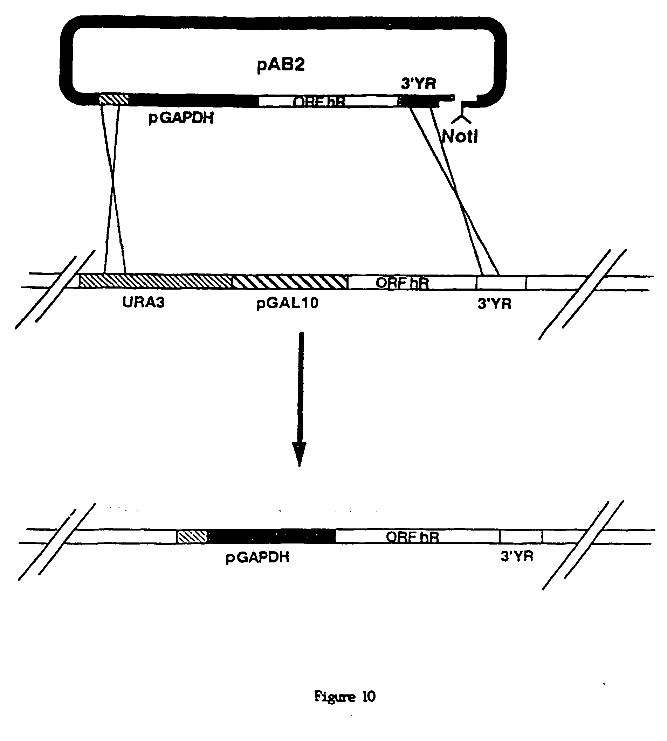

Figure 10: construction de la souche W(GhR,ΔB) avec pAB2.

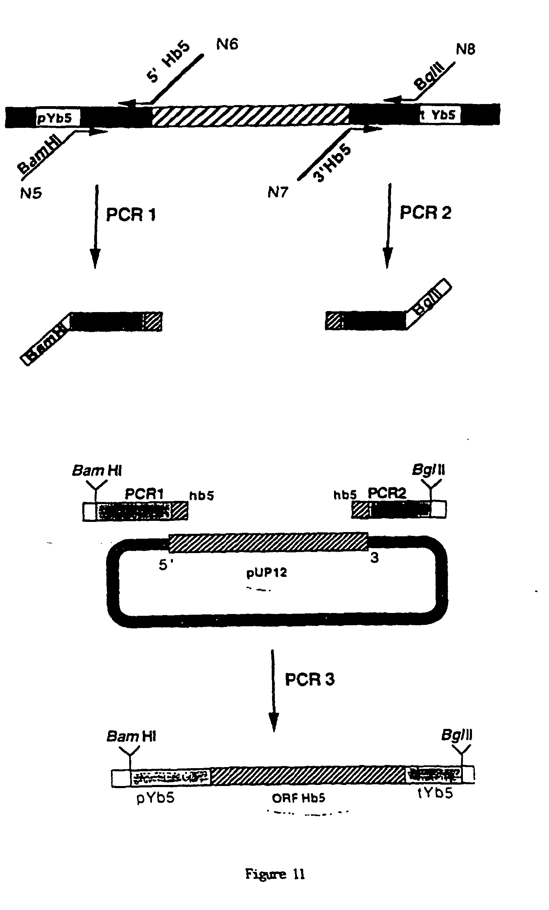

Figure 11: construction de la cassette hb5.
Figure 12: construction de pAB3.

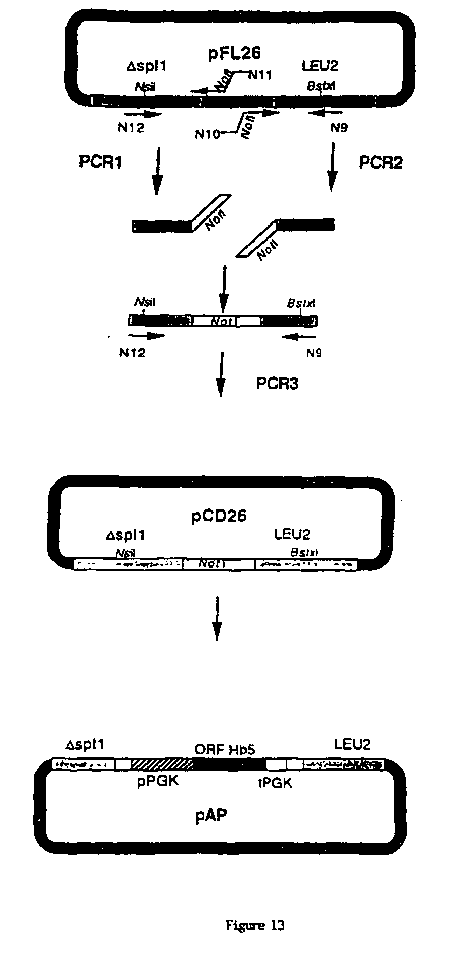

Figure 13: construction de pAP1.

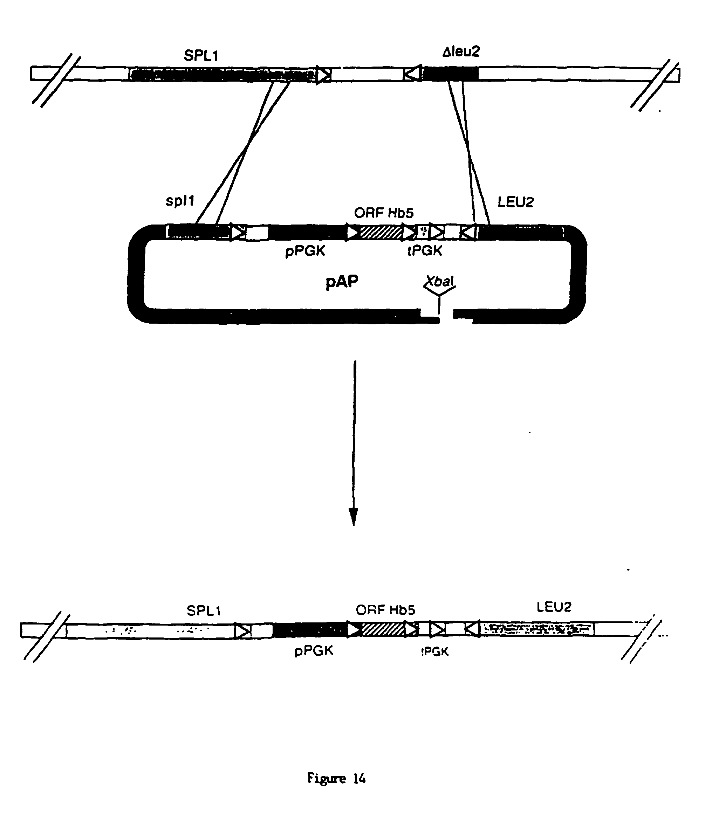

Figure 14: construction de la souche W (GhR, Lhb5).

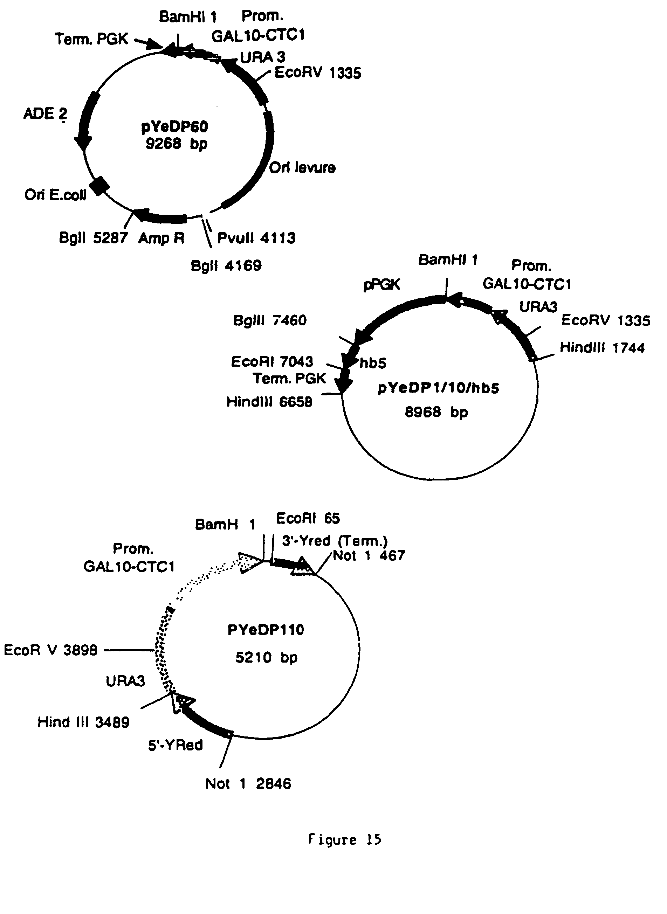

Figure 15: Représentation des plasmides pYeDP60, pYeDP1/10/hb5 et pYeDP110.

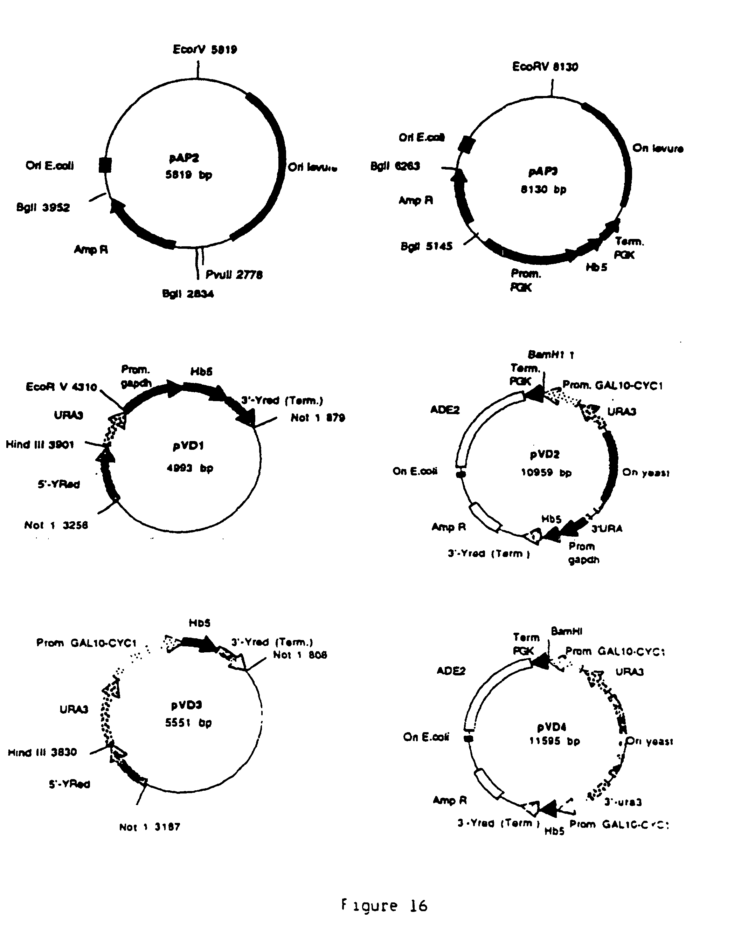

Figure 16: Représentation des plasmides pAP2, pAP3, pVD1, pVD2, pVD3, pVD4.

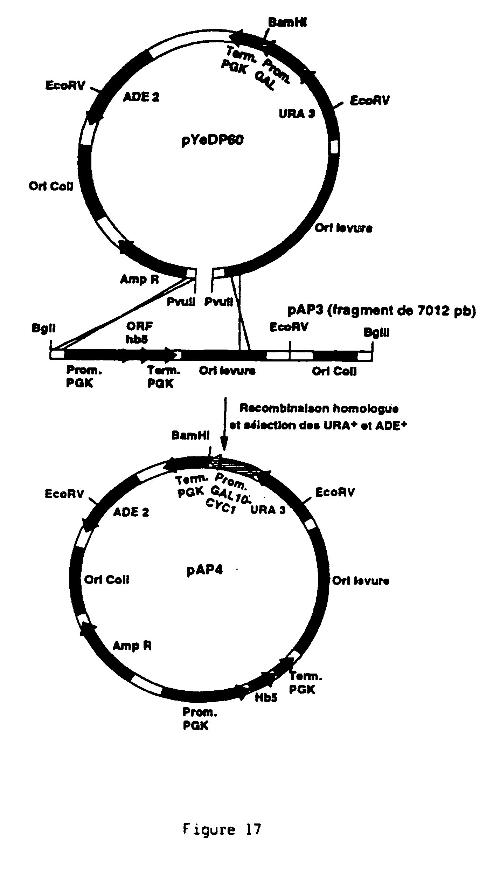

Figure 17: Construction et structure de pAP4.

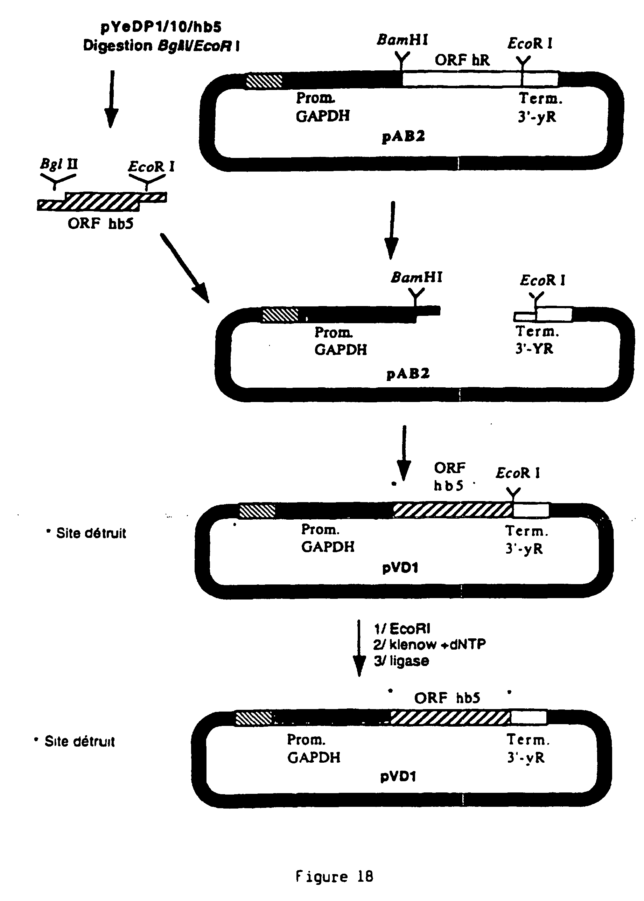

Figure 18: Construction de pVD1.

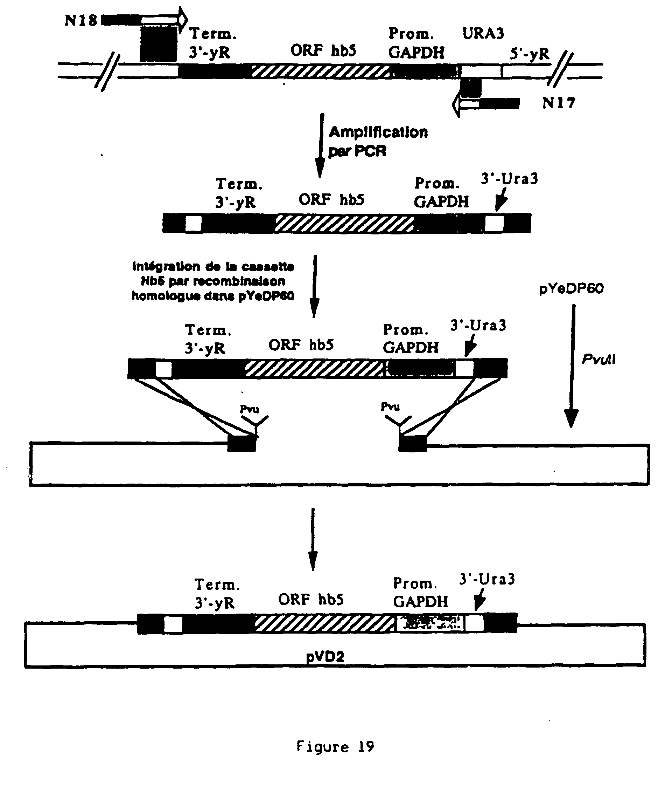

Figure 19: Construction de pVD2.

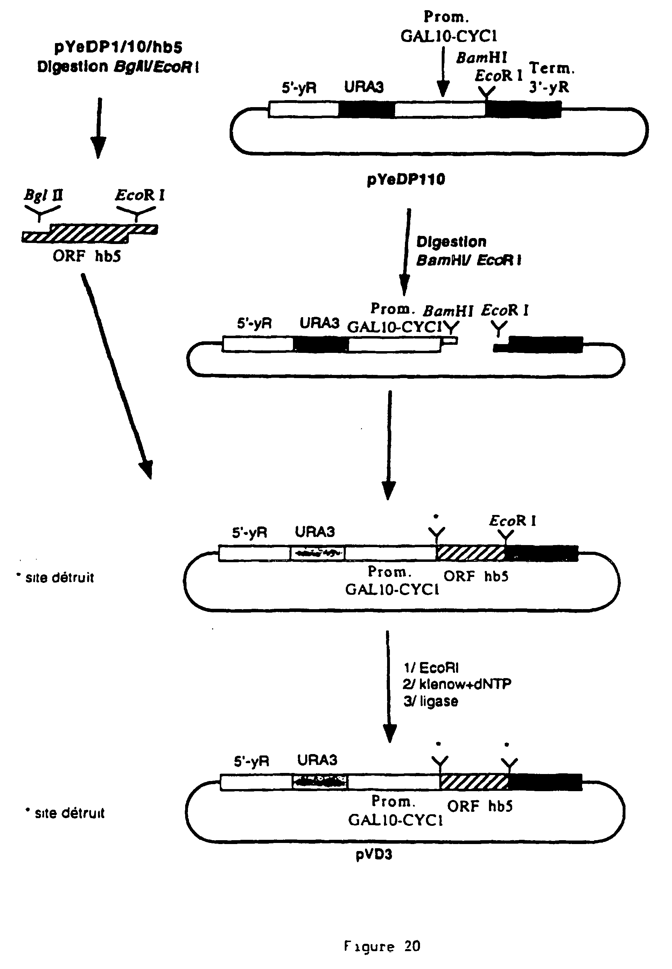

Figure 20: Construction de pVD3.

### MATERIELS ET METHODES:

### 1- Milieux:

Voir tableau I page suivante.

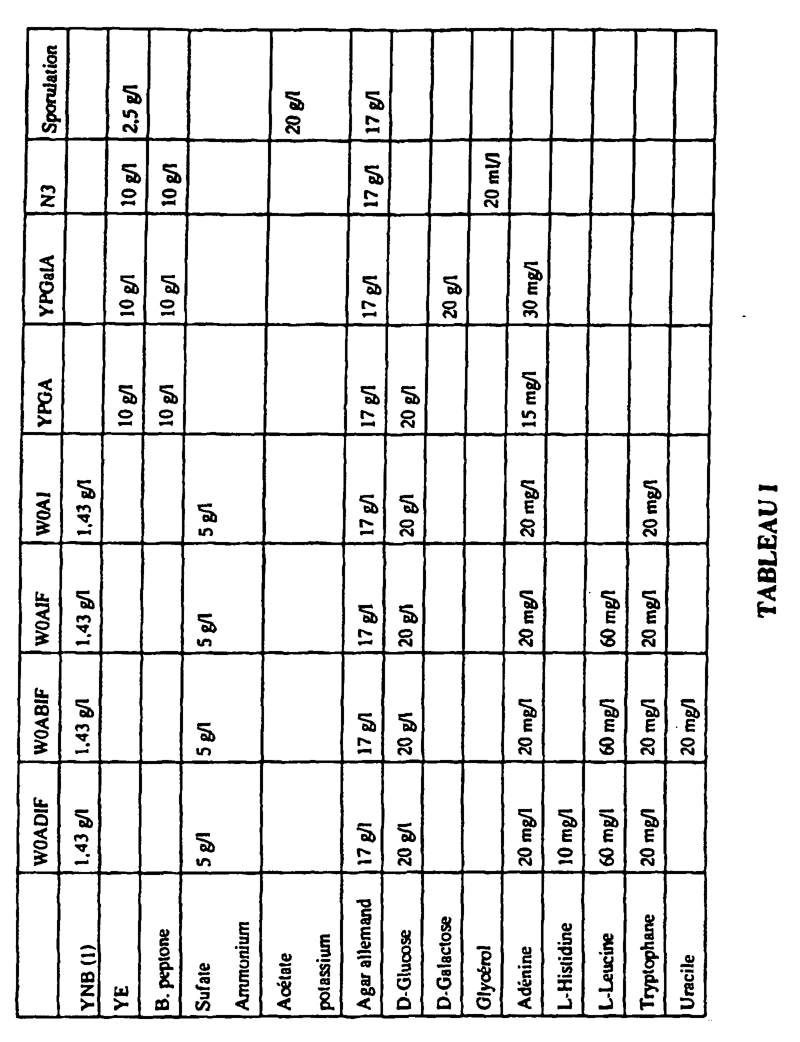

Le milieu de sporulation est complété de manière à complémenter les différentes auxotrophies suivant les souches.
(1) Le yeast nitrogen base (YNB) sans acides aminés et sans ammonium provient de chez GIBCO BRL. Le sulfate d'ammonium de chez MERCK. L'agar allemand de chez ROHSTOFF Gmbh. L'adénine, le L-histidine et le L-tryptophane de chez SIGMA. La L-leucine et l'uracile de chez CALBIOCHEM. Le D-glucose, le D-galactose, le glycérol et l'acétate de potassium anhydre de chez PROLABO. Le bactopeptone(B. peptone) et le yeast extract (YE) de chez DIFCO.
Le N3 est tamponné avec un tampon phosphate à pH=6.2 obtenu en dissolvant 89 g de Na₂HPO₄, 272 g de KH₂PO₄ et 1 g de specilline dans 5 l d'eau.
Les milieux liquides ont la même composition que les milieux solides sauf qu'ils ne contiennent pas d'agar.
Ringer: 0,9 % NaCl dans de l'eau.

### 2- Souches:

- Levure : *S. cerevisiae:*
   W(N): *MAT*a et *a, leu2*-3,112, *his3*-11, *ade2*-1, *trp1*-1, *ura3-*1*.* W(N)a désigne W(N) *Mat a* et W(N)a désigne W(N) *Mat a.*
   W(ΔB): *MATa* et *a, leu2-*3,112, *ade2*-1*, trp1*-1*, ura3*-1*,* Yb5: HIS3. W(ΔB)a désigne W(ΔB) *Mat a* et W(ΔB)a désigne W(ΔB) *Mat a.*
   W(hR): MATa et a, *leu2*-3,112, *his.3*-11*, ade2*-1*, trp1*-1*,* YRED: [GAL10-CYC1::HRED]. W(hR)a désigne W(hR) *Mat a* et W(hR)a désigne W(hR) *Mat a*
- Bactérie:
   *E. coli* DH5-1: supE44, hddR17, recA1, gyrA96, thi-1, relA1.

### 3- Les vecteurs:

- Plasmide pUP81 : la phase codante du gène de la cytochrome P450 réductase humaine obtenue par PCR avec les primers N1 et N2, est coupée par *Bam*HI et *Bgl*II, puis clonée dans le vecteur d'intégration DP110 au niveau de son site *Bam*HI, l'ATG du côté du promoteur GAL10-CYC1 (Urban et al., 1993).
   Primer N1: 5'-GCggatccATGGGAGACAGTCACGTGG-3'(SEQ ID n°1), les bases 1 à 2 forment un GC clamp, les bases 3 à 8 (lettres minuscules) correspondent au site *Bam*HI ajouté, les bases 9 à 17 et 21 à 27 sont respectivement homologues aux nucléotides 1 à 9 et 13 à 19 de la séquence de la phase ouverte de lecture du gène de la réductase humaine, les bases 18 à 20 (en caractères gras) permettent de muter les nucléotides TCC en position 10 à 12 à partir de l'ATG de la séquence codante de la réductase humaine. Cette mutation permet de détruire la structure en fourche de l'ARN transcrit dans les 20 premières paires de bases, responsable de l'inlubition de la traduction chez la levure (Baim et al., 1988).
   Primer N2: 5'-CGgaattcAGATCTAGCTCCACACGTCCAGG-3'(SEQ ID n°2), les bases 1 à 2 forment un GC clamp, les bases 3 à 8 (lettres minuscules) correspondent au site *EcoR*I*,* les bases 9 à 14 (lettres soulignées) correspondent au site *Bgl*II*,* les bases 14 à 16 (caractères gras) correspondent au codon stop (brin complémentaire) et les bases 13 à 31 sont complémentaires aux nucléotides 2018 à 2034 de la phase ouverte de lecture du gène de la réductase humaine.
- Plasmide pFL26 (Bonneaud et al., 1991).
- Plasmide pPL100: ADE2/pFL (Stotz & Linder). Le gène ADE2 de levure a été modifié afin de construire le vecteur pPL100. Le site *Bgl*II interne du gène est détruit en mutant l'adénosine en position 593, à partir de l'ATG, en guanine. Cette mutation ne change ni la séquence en acides aminés ni l'activité protéique. Un site *Bgl*II est introduit dans la région 5'- du gène ADE2 en position -373 à partir du codon d'initiation. Le gène possède un site *Bgl*II en position 1862 à partir de l'ATG. Le fragment *Bgl*II de 2241 pb est cloné dans le vecteur pFL36 au site *Bgl*II (Bonneaud et al.,1991).
- Plasmide "Blue-Script" est décrit dans le kit "pCR-Script™ SK(+) cloning kit" (Stratagène).

### 4- Croisement:

Les souches haploïdes de signes opposés (a ou a), sont cultivées séparément en milieu complet YPGA. Les cellules sont ensuite diluées dans du Ringer à 10⁵ cellules/ml environ. Un mélange de 500 ml de chacune des deux suspensions de cellules haploïdes est préparé. A partir du mélange, 50 ml de suspension sont étalés sur milieu solide YPGA. Au bout de 8 h de croissance, les cellules sont sous-cionées sur un milieu sélectif qui ne complémente que les auxotrophies présentes simultanément dans les deux parents. Ceci permet la croissance du diploïde issu du croisement, mais pas des haploïdes d'origine. Au bout de deux jours de croissance, les clones diploïdes qui apparaissent sont repiqués sur le même milieu sélectif.

### 5- Sporulation:

Les cellules diploïdes sont repiquées sur un milieu de sporulation solide complémenté par les marqueurs d'auxotrophie du diploïde. Au bout de 3 jours de sporulation, les spores sont disséquées.

### 6- Dissection des spores:

### Technique dite en vrac:

les spores sont diluées dans des tubes Eppendorff de 1,5ml, à 5.10⁸ cellules/ml, dans de l'eau contenant de la zymolyase 10.000 (Seikagaku Kogyo Co., Tokyo, Japan) à 100 mg/ml, puis incubées pendant 30 min à 28°C. Un aliquot de 500 ml est centrifugé à 10.000 rpm pendant 1 min. Le culot cellulaire est repris dans 1 ml d'eau, centrifugé et repris dans 100 ml d'eau. Les cellules sont agitées au vortex pendant 2 min. Le tube est rincé à l'eau en le retournant 2 à 3 fois. Au bout de 5 rinçages, les spores qui sont hydrophobes, adhèrent aux parois du tube, alors que les cellules végétatives diploïdes restent en suspension et sont éliminées par rinçage. Les spores sont ensuite resuspendues dans une solution de 0.01 % (v/v) Nonidet P-40 (Sigma). Le détergent est éliminé après centrifugation. Les spores purifiées sont reprises dans du Ringer et étalées sur milieu sélectif solide.

### Microdissection:

Les spores sont incubées dans une solution (à 55 % glycérol) de cytohélicase (Biosepra) à 0,5 g/l pendant 15 min à 22°C, puis étalées sur un morceau d'agar préalablement découpé et déposé sur une lamelle. La lamelle est ensuite déposée à l'envers sur une chambre de microdissection. Les quatres spores, contenues dans une tétrade, sont séparées sous microscope à l'aide du micromanipulateur. L'agar, supportant les tétrades ainsi disséquées, est déposée sur une boite de milieu complet YPGalA.

### 7. Clonage du promoteur GAPDH:

### Amplification par PCR:

L'ADN du promoteur du gène de la glycéraldéhyde phosphodéshydrogénase de levure a été cloné par la technique de la PCR à partir de 100 ng d'ADN génomique de *S. cerevisiae* W(N) préparés suivant la méthode décrite (Bellamine et al., 1994), en utilisant comme amorces 50 ng des primers N3 (SEQ ID n°3) et N4 (SEQ ID n°4) (Fig 9). L'amplification est faite avec 2,5 U de *Pfu* ADN polymérase native (Stratagène). La réaction de polymérisation a lieu dans 50 ml d'une solution 20 mM Tris-HCl, 10 mM KCl, 6 mM (NH₄)₂SO₄, 2 mM MgCl₂ 0,1 % Triton X-100, 10 mg/ml de "serum albumin" sans nucléase et 200 mM de chacun des quatre désoxynucléotidetriphosphates (dNTP). Les conditions de PCR sont les suivantes:

| 2 précycles | 95°C | 10 sec |
|---|---|---|
| | 40°C | 50 sec |
| | 60°C | 5 sec |
| | 74°C | 2 min |

| 25 cycles | 95°C | 5 sec |
|---|---|---|
| | 48°C | 50 sec |
| | 65°C | 5 sec |
| | 74°C | 2 min |

Les séquences des primers sont:
- Primer N3: 5'-CCaagcttGAGTTTATCATTATCAATACTCG-3'(SEQ ID n°3), les deux premières bases forment un GC clamp, les lettres en minuscules correspondent au site *Hind*III*,* les bases allant de 9 à 31 sont homologues aux nucléotides -673 à -650 de la séquence du promoteur du gène GAPDH.
- Primer N4:
   5'-CggatccTATTTATGTGTGTTTATTCGAAACTAAGTTCTTGG-3'(SEQ ID n°4), les lettres en minuscules correspondent au site *Bam*HI, les bases allant de 8 à 42 sont complémentaires aux nucléotides -6 à -48.

La taille de l'ADN amplifié est de 691 pb.

26 ml d'acétate d'ammonium 7,5 M et 4 ml d'eau sont rajoutés au 50 ml du produit PCR. Le mélange est précipité par 80 ml d'éthanol pendant 10 min à 22°C. L'ADN précipité est centrifugé à 10.000 rpm pendant 10 min. Le culot est lavé à l'éthanol 70 % (v/v), séché, et repris dans 20 ml d'eau.

Un aliquot de 10 ng de cet ADN est cloné au site *Srf*I du vecteur Blue Script du kit "pCR-Script™ SK(+) cloning kit" suivant les recommandations du vendeur (Stratagène). Les clones sont triés par restriction *Pvu*II. Ceux qui donnent des fragments *Pvu*II à 2513 et 1139 pb, sont séquencés sur 100 pb au niveau de la jonction entre l'ADN cloné et le vecteur Blue-Script avec le kit: Sequenase Version 2.0 DNA Sequencing Kit U. S. B. (Amersham). Ces constructions sont appelées pAB1.

Clonage du promoteur GAPDH dans le vecteur d'intégration pUB81:
le clone pAB1 est coupé par *Hind*III. Cette coupure génère un site bout collant qui est rempli grâce au fragment de *Klenow* de la DNA polymérase (Biolabs). Le vecteur pAB1 ainsi linéarisé (fragment de 3652 pb) est ensuite coupé, à son extrémité 5'par *Bam*HI puis cloné dans le vecteur pUP81 entre les sites *Eco*RV et *Bam*HI. Lors de la ligation, le site *Hin*dIII rendu bout franc, s'adapte au site *Eco*RV qui est bout franc, alors que les deux demi-sites *Bam*HI du fragment pGAPDH et du vecteur d'intégration pUP81, se lient entre eux. Le site *Eco*RV a été choisi pour disrupter en même temps le gène URA3 (Fig 9). Les clones sont triés par restriction enzymatique avec les enzymes *Pst*I et *Bam*HI. Ceux qui donnent des fragments d'ADN de 600 et 800 pb en plus des fragments 60, 104, 197, 385, 440 et 2564 pb (fragments qui existent dans le vecteur pUB81), représentent les vecteurs pUP81 contenant l'ADN du promoteur GAPDH. Cette construction est appelée pAB2. Trois clones contenant le plasmide pAB2 sont vérifiés par séquençage sur 100 pb avec le primer N3(SEQ ID n°3) à partir de l'extrémité 5'de l'ADN du promoteur du gène GAPDH.

### 8- Construction de la cassette d'intégration du cytochrome b5 humain au locus YCYB5:

Cette construction a été faite en trois PCR différentes.

Dans la première PCR (PCR 1), les 353 pb de l'extrémité 3'du promoteur du gène du cytochrome b5 de levure (Yb5), ont été amplifiées en utilisant les primers N5 (SEQ ID n°5) et N6 (SEQ ID n°6) et l'ADN génomique de levure W(N):
- Primer N5: 5'-ggatccGAGCGGGTAATAGCCTGGAGTTTCC-3'(SEQ ID n°5), comporte un site *Bam*HI à partir de son extrémité 5'(en lettres minuscules). Ce primer est homologue, de la base 7 à 31, aux nucléotides -331 à -306 de la phase ouverte du promoteur du gène du cytochrome b5 de levure.
- Primer N6:
   5'-ccgactgctctgccatGATTGTTTGATATTTTATGTTGTAGTTGATTG-3'(SEQ ID n°6), comporte à partir de son extrémité 5': base 1 à 16 la séquence complémentaire aux 16 premiers nucléotides de l'extrémité 5' de la phase ouverte de lecture du gène du cytochrome b5 humain (lettres minuscules), base 17 à 49 la séquence complémentaire aux nucléotides -1 à -32 du promoteur du gène du cytochrome b5 de levure.

Le fragment amplifié fait 375pb.

Dans la deuxième PCR (PCR 2), les 147 dernières pb de la partie 5'du terminateur du gène du cytochrome b5 de levure ont été amplifiées par les primers N7 (SEQ ID n°7) et N8 (SEQ ID n°8):
- Primer N7:
   5'-cctatacatggcagaggactgaATTCTITITCTTCCAGAATAGCCCACAC-3'(SEQ ID n°7), comporte à partir de son extrémité 5': base 1 à 22: séquence homologue aux nucléotides 383 à 405 de la phase ouverte du gène du cytochrome b5 humain (lettres minuscules), base 23 à 50 la séquence homologue aux nucléotides 1 à 28 du terminateur à partir du codon stop.
- Primer N8: 5'-GGagatctGTGACATACTTCTATGCGATATAG-3'(SEQ ID n°8), comporte de la base 1 à 2 un GC clamp et un site *Bgl*II de la base 3 à 8 (en lettres minuscules). Ce primer est complémentaire, de la base 9 à 32, aux nucléotides 1 à 147 de la phase ouverte du terminateur du gène YCYB5 de levure à partir du codon stop.

Le fragment amplifié fait 180 pb.

Dans les PCR 1 et 2, l'ADN génomique de la levure W(N) est utilisé comme matrice.

Dans une troisième PCR (PCR 3), 4 amorces sont utilisées:
- Deux cents nanogramme des produits des deux premières PCR (375 et 180 pb) sont utilisés comme amorces et 100 ng d'ADN de la phase codante du cytochrome b5 humain comme matrice (Fig 11). Ceci permet d'avoir la cassette d'intégration du b5 humain (CIH) mais en faible quantité.
- Le produit de cette PCR est ensuite amplifié par les amorces N5 (SEQ ID n°5) et N8 (SEQ ID n°8), ceci a permis d'avoir la cassette d'intégration en plus grande quantité. Le produit de fusion est traité après amplification à la *Pfu* polymérase en présence de dNTP 0,2 mM pendant 30 min à 76°C. Le fragment obtenu fait 917 pb. Les amplifications sont faites à la Taq polymérase (Appligène).

Les programmes PCR utilisés sont les suivants:

**PCR 1 et PCR 2:**

| 2 précycles | 88°C | 3 sec |
|---|---|---|
| | 95°C | 10 sec |
| | 42°C | 2 min |
| | 60°C | 3 sec |
| | 74°C | 2 min 30 sec |

| 30 cycles | 88°C | 3 sec |
|---|---|---|
| | 95°C | 5 sec |
| | 50°C | 1 min |
| | 65°C | 3 sec |
| | 74°C | 2 min. |

**PCR 3:**

| 3 précycles | 88° | 3 sec |
|---|---|---|
| | 95°C | 10 sec |
| | 40°C | 5 min |
| | 60°C | 3 sec |
| | 74°C | 3 min |

| 15 cycles | 88°C | 3 sec |
|---|---|---|
| | 95°C | 5 sec |
| | 45°C | 2 min |
| | 65°C | 3 sec |
| | 74°C | 2 min. |

La cassette d'intégration ainsi construite (CIH), est clonée dans le vecteur pCRScript au niveau du site *Sfr*I. Le clone obtenu pLIP1 est contrôlé par séquençage sur 200 pb au niveau des deux jonctions: du promoteur et du terminateur du gène YCYB5 de levure et de la phase codante du gène du cytochrome b5 humain . Afin de ralonger les pieds de recombinaison pour que l'intégration de la cassette d'intégration du b5 humain se fasse correctement, une nouvelle cassette d'intégration comportant la phase codante du gène du cytochrome b5 humain et la totalité du promoteur et du terminateur du gène YCYB5 de levure a été construite à partir du fragment *Bam*HI/*Bg*lII de 917 pb du vecteur pLIP1. Le vecteur YCYB5/YEP352 qui contient la totalité du gène YCYB5 de levure (Truan et al., 1994), est coupé à un site unique *Cla*I*.* Ce fragment linéarisé est cotransformé dans la souche W(N)a avec le fragment *Bam*HI/*Bg*lII de 917 pb du vecteur pLIP1. La linéarisation du vecteur YCYBS/YEP352, étant au niveau de la phase codante du gène YCYB5, le cytochrome b5 de levure sera substitué par le cytochrome b5 humain à la suite d'une recombinaison homologue dans la levure (Fig 12). La sélection des recombinants se fait grâce à la recircularisation du vecteur d'expression (Bellamine et al., 1994). Le nouveau vecteur pAB3 est récupéré à partir de la levure puis amplifié dans la bactérie *E. coli* selon la méthode décrite (Bellamine et al., 1994).

### 9- Construction de la cassette d'intégration du cytochrome b5 humain au site intergénique leu2D/SPL1:

Une cassette permettant d'intégrer le gène du cytochrome b5 humain à proximité immédiate du gène LEU2 dans la région intergénique avec 500 pb du gène SPL1 a été construite en deux étapes:
- Construction du vecteur pCD26: à partir du vecteur pFL26 (Bonneaud et al., 1991) portant à la fois le gène LEU2 et 500 pb du gène SPL1, un site *Not*I a été introduit en trois PCR (Fig13).
Dans la première PCR, les 704 pb de la partie 3'- du gène LEU2 sont amplifiées en utilisant les primers N9 (SEQ ID n°9) et N10 (SEQ ID n°10):
- Primer N9: 5'-TTGAAGGTTCAACATCAATTGATTG-3 (SEQ ID n°9)' est homologue aux nucléotides 2190 à 2214 du vecteur pFL26 qui correspondent à la fin de la phase ouverte du gène LEU2 (la numérotation, au niveau du vecteur pFL26, est faite à partir du nucléotide 1 placé à 417 pb en amont du site *Bam*HI*).*
- Primer N10: 5'-GTGTGgcggccgcCTCCTTGTCAATATTAATGTTAAAG-3'(SEQ ID n°10), comporte à son extrémité 5'-, cinq bases complémentaires aux nucléotides 2890 à 2894 du vecteur pFL26, un site *Not*I de la base 6 à 13 et une séquence complémentaire des nucléotides 2857 à 2881 de la base 14 à 38.
Dans la deuxième PCR, les 347 dernières pb de la partie 3'du gène SPL1 sont amplifiées en utilisant les primers N11 (SEQ ID n°11) et N12 (SEQ ID n°12):
- Primer N11: 5'-CAAGGAGgcggccgcCACACAAAAAGTTAGGTGT-3'(SEQ ID n°11), comporte à son extrémité 5', sept bases complémentaires des nucléotides 2875 à 2881 de la séquence du vecteur pFL26, un site *Not*I de la base 8 à 15 et une séquence homologue aux nucléotides 2889 à 2908 du pFL26, de la base 16 à 34.
- Primer N12: 5'-TCTGCTTCCCTAGAACCTTCTTATG-3'(SEQ ID n°12) est complémentaire des nucléotides 3198 à 3222 de l'extrémité 3'du brin codant pour la phase ouverte du gène SPL1 dans le vecteur pFL26.
Dans la troisième PCR, 100 ng des deux fragments issus des deux premières PCR (ayant 20 pb de chevauchement qui contiennent le site *Not*I*),* sont utilisés comme matrice et les primers N9 et N12 comme amorces. Les 1031 pb amplifiées sont clonées dans le vecteur pFL26 aux sites *Nsi*I et *Bst*XI pour donner le vecteur pCD26.
Les amplifications sont faites à la Taq polymérase Appligène. Les deux premières PCR utilisent le même programme que les PCR 1 et 2 du paragraphe 8, la PCR 3 utilise le programme suivant :

| 30 cycles. | | |
|---|---|---|
| | 95°C | 10 sec |
| | 60°C | 5 sec |
| | 45°C | 1 min |
| | 65°C | 5 sec |
| | 74°C | 2 min |

- Construction du vecteur d'intégration pAP1: A partir du plasmide pUP12 préalablement construit (Urban et al., 1990), la cassette d'expression du cytochrome b5 humain sous le contrôle du promoteur et du terminateur du gène PGK (phosphoglycérate kinase) de levure, est récupérée grâce à une coupure *Bam*HI/*Hind*III. Ce fragment de 2400 pb est rendu bouts francs grâce à la Mung Bean nucléase (Biolaps), puis cloné dans le vecteur pCD26 coupé au site *Not*I rendu bout franc grace au fragment de *Klenow* de la DNA polymérase (**Fig 13**). Les clones qui donnent des fragments de restriction *Pst*I de 5154 et 2904 pb dénommés pAP1 sont utilisés pour l'intégration.

### 10- Vecteurs de transformation par cytochrome P450

Le plasmide pYeDP60 est un vecteur navette (bactérie, levure) de 9265 pb possédant les origines de réplication *ori E. coli* et *ori* 2 µ, le gène *bla* qui code pour la résistance à l'ampicilline, les gènes *URA3* et *ADE 2* qui sont des marqueurs de complémentations d'auxotrophie, un promoteur hybride *GAL10-CYC1* inductible en présence de galactose et le terminateur de transcription du gène *PGK.* Un polylinker comprenant entre autres les sites de restriction *Bam*HI, *Kpn*I, *Eco*RI est inséré entre promoteur et terminateur de transcription à fin de clonage d'un ADNc à exprimer.

Les plasmides 1A1/V60 et 3A4/V60 correspondent au vecteur V60 dans lequel les phase codante des ADNc codant respectivement pour les cytochromes P450 1A1 et 3A4 humains ont été insérés sous contrôle du promoteur *GAL10-CYC1* et du terminateur de transcription *PGK.*

### 11. Milieu de culture pour la transformation des souches de levure.

Milieu YPGE
- extrait de levure 10 g/l
- bactopeptone 10 g/l
- glucose 5 g/l
- éthanol 30 ml/l

Milieu SW6:
- base azotée de levure(Difco) 7g/l
- Glucose 20 g/l
- Casaminoacide (Difco) 1g/l
- Tryptophane 20 mg/l
- Agar 15 g/l (pour les milieux solides uniquement).

Pour le milieu galactosé correspondant (SW5) le glucose est remplacé par le galactose à la même concentration.

### 12- Préparations des fractions microsomales.

### 12.1 Transformation.

Les levures sont transformées par les plasmides 1A1/V60 ou 3A4/V60 selon la méthode standard dite au "chlorure de lithium". Les transformants sont sélectionnés sur un milieu de culture de levure glucosé synthétique dépourvu d'adénine et d'uracile (SW6) auquel est additionné les nutriments nécessaires pour complémenter les auxotrophies résiduelles selon la souche utilisée (voir génotype des souches).Une forte proportion des transformants primaires sélectionnés sur le milieu glucosé ne poussent pas sur un milieu minimum de culture galactosé. Seuls les clones qui se développent correctement sur milieu galactose sont conservés

### 12.2 Préculture:

les clones sélectionnés sont repiqués sur milieu minimum non inducteur glucosé sélectif pour le plasmide (généralement milieu SW6), puis incubés une nuit à 28 °C, dans 20 ml du même milieu liquide.

### 12.3 Culture:

250 ml de milieu YPGE sont ensemencés avec la préculture et incubés sous agitation à 28 °C jusqu'à obtenir une densité cellulaire comprise entre 8 et 9,6 10⁷ cellules/ ml. Du galactose est ensuite rajouté à une concentration de 20 g/l, et la culture est incubée à 28°C pendant la nuit, de façon à obtenir une densité cellulaire de 2 10⁸ cellules/ml. La présence de galactose permet l'induction du cytochrome P450 et des autres gènes dépendant du promoteur GAL10-CYC1.

### 12.4 Fractionnement des cellules:

les cellules sont centrifugées et lavées dans du tampon TE pH 7,4 (Tris-HCl, 50 mM; EDTA 1 mM); KCl 0,1 M, et remises en suspension dans du tampon TE pH 7,4; Sorbitol 0,6 M. Afin de casser les cellules, des billes de verre sont ajoutées et les tubes sont vigoureusement agités de bas en haut pendant 5 min à 4°C. Les étapes suivantes sont effectuées à 4°C. La suspension de cellules cassées est récupérée et les billes sont lavées plusieurs fois avec le même tampon. Afin d'éliminer les débris cellulaires, les noyaux ainsi que la fraction mitochondriale, deux centrifugations respectivement de 3 min à 3500 rpm et 10 min à 15 000 rpm sont effectuées. Pour précipiter les microsomes, le surnageant est incubé en présence de NaCl (0,15M final) et de PEG 4000 (10% final) pendant 15 min dans la glace. Après une centrifugation de 10 min à 10000 rpm le culot de microsomes est récupéré dans du tampon TE pH 7,4 ; 20 % glycérol. La préparation de microsomes est aliquotée et stockée à -80°C.

### 13- Dosage du cytochrome P450

La concentration en cytochrome P450 dans les fractions microsomales est déterminée spectralement. Le cytochrome P450 à doser est dilué dans du tampon TE pH 7,4 (environ 1 mg de protéines microsomales par ml) et réduit par quelques grains de dithionite de sodium. Après enregistrement de la ligne de base (cytochrome P450 réduit contre cytochrome P450 réduit), quelques bulles de CO sont ajoutées à la cuve de mesure et le spectre différentiel est mesuré entre 400 et 500 nm. Le CO forme un complexe stable avec le fer réduit du cytochrome P450, ce complexe présente un maximum d'absorption caractéristique à 450 nm. Le coefficient d'absorption _εM (450-490 nm) est de 91 mM⁻¹. cm⁻¹.

### 14- Dosage des protéines microsomales

Le dosage des protéines totales est réalisé avec le kit de dosage PIERCE-BCA dans les conditions données par le fabricant. La sérum albumine bovine est utilisée comme standard.

### 15- Activités catalytiques des cytochromes P450

### 15.1 Tests d'activité EROD sur microsomes

Le cytochrome P450 1A1 catalyse la O-deséthylation de la 7-ethoxyrésorufine. Le produit de la réaction, la résorufine (7-hydroyphénoxasine), après excitation à 530 nm, fluoresce à 586 nm. La quantité de résorufine formée par unité de temps correspond à la vitesse d'accumulation de la résorufine.

Le mélange d'incubation comprend:
- 2 µl d'une suspension microsomale (entre 20 et 50 µg de protéines microsomales) dans 1 ml de tampon TE pH 7,4, contenant du NADPH 50 µM et 2,5 µM de 7-éthoxyrésorufine. Pour déterminer l'effet du cytochrome b5 de lapin sur l'efficacité catalytique, les fractions microsomales sont préalablement incubées au froid en présence d'un excès de cytochrome purifié.

### 15.2 Test d'activité THL sur microsomes: 6β-hydroxylation de la testostérone

La testostérone est une hormone stéroïde hydroxylée en position 6β par le cytochrome 3A4. Le milieu d'incubation comprend 100 µg de protéines microsomales dans 0,25 ml de tampon Tris 50 mM, 1 mM EDTA pH 7,4 ou phosphate de sodium 50 mM pH 7.4, contenant du NADPH 50 µM, de la testostérone 80 µM (à partir d'une solution mère 5 mM dans l'éthanol), en absence ou en présence d'un excès de cytochrome b5.

Les incubations ont lieu pendant 10 min à 28°C ou 37°C. La réaction est arrêtée par addition de 10 µl de TFA 50% dans l'eau. La procédure d'extraction est la suivante:
- addition de 500 µl de dichlorométhane
- agitation au vortex à la vitesse maximale pendant 1 min
- centrifugation pendant 5 min à 10 000rpm
- élimination de la phase aqueuse supérieure
- évaporation de la phase organique sous flux d'azote.

Le résidu sec est repris avec 20 µl de méthanol, puis 20 µl d'eau sont ajoutés. La moitié est injectée dans une colonne HPLC de phase inverse SPHERI-5RP-18,5 µm (100 x2,1 mm) en utilisant comme éluant l'acétonitrile à 1 ml/min. La composition en acétonitrile du gradient d'élution varie de 10% (vol/vol) à 0 min jusqu'à 60% à 8 min. La détection est effectuée à 254 nm. Les temps d'élution sont 6 min 20s pour la β-hydroxytestostérone et 8 min pour la testostérone.

### EXEMPLES

### Exemple n°1 : Construction de la souche W(hR, ΔB):

La souche W(ΔB)a (qui pousse sur milieu W0ABIF) et la souche W(hR)a (qui pousse sur milieu W0ADIF) sont croisées entre elles et le diploïde est sélectionné sur milieu glucose WOAIF. Ce milieu est létal pour chacun des haploïdes et non pour le diploïde W(hR/YR, Yb5/DYb5). Les clones diploïdes sont sous-clonés sur le même milieu sélectif. Après sporulation, les tétrades sont disséquées soit par microdissection soit par dissection en vrac. Les spores cultivées sur milieu YPGalA sont ensuite repiquées sur différents milieux sélectifs contenant du galactose comme source de carbone, afin de tester les auxotrophies des différentes spores. Les clones de levure qui poussent sur milieu galactose et qui sont prototrophes pour l'uracile et l'histidine correspondent à la souche W(hR, ΔB). Ces levures ne poussent plus quand on remplace sur le même milieu le galactose par le glucose car, dans ces conditions, la réductase humaine ne s'exprime pas. Ces souches étant alors déficientes à la fois en réductase et en b5 de levure (dont le gène est disrupté), ne poussent pas car la double déficience est létale (Truan et al., 1994). Quatre clones sont retenus pour la suite: Sp1 et Sp2 obtenus par microdissection, et C1 et C2 obtenus par dissection en vrac.

### Exemple n°2 : Construction de la souche W(GhR, ΔB):

Le vecteur d'intégration du promoteur GAPDH pAB2 coupé par *NotI,* est utilisé pour transformer la souche W(hR, ΔB)a. Le vecteur pPL100 est utilisé comme marqueur de cotransformation (10 à 15 ng d'ADN du vecteur pPL100 pour 2 mg d'ADN du vecteur pAB2). Les transformants sont sélectionnés sur milieu WOBDIF. Les clones passent par 3 séries de cribles:
- Une sélection de l'auxotrophie à l'uracile: les transformants sont repiqués sur milieu W0ABDIF, puis sur milieu W0ADIF pour repérer les clones qui ne poussent pas en absence d'uracile. La perte du gène URA3 suggère que le promoteur GAPDH a remplacé le promoteur GAL10-CYC1, car l'intégration du promoteur GAPDH à la place du promoteur GAL10-CYC1 inactive en même temps le gène URA3 qui est en amont du promoteur. 25% des transformants ne poussent pas en absence d'uracile (Fig 10).
- La deuxième sélection: pour vérifier l'activité de la réductase humaine exprimée sous le contrôle du promoteur GAPDH, la résistance au kétokonazole des clones W(GhR, AB) a été évaluée (Brevet n°WO94/01564). Trois clones différents ont une résistance au kétokonazole de 20 mg/ml alors que la souche W(hR), dans laquelle la réductase humaine est exprimée sous le contrôle du promoteur GAL10-CYC1, a une résistance de 1 à 5 mg/ml dans les mêmes conditions .
- Troisième sélection: afin d'estimer le niveau d'expression de la réductase humaine dans la souche W(GhR, ΔB), la réduction du cyctochrome *c* par la réductase contenue dans les fractions microsomales des trois clones étudiés est mesurée (Truan et al., 1993). Suivant les conditions de culture, la réduction du cytochrome c est de 1,5 à 3 fois plus importante que la souche W(hR).
- Test de respiration: les clones obtenus sont repiqués sur milieu solide N3 afin de tester leur phénotype respiratoire. Les trois clones ne poussent pas sur N3. Ils sont donc phénotype respiration négative. Pour les rendre [respiration positive], ces clones sont croisés avec une souche de phénotype respiration positive W(hR)a. Le diploïde obtenu W(GhR/hR, ΔB/Yb5) est sélectionné sur milieu WOAIF. Après sporulation et dissection en vrac, l'ADN génomique des haploïdes est préparé puis une PCR utilisant les primers N13 (SEQ ID n°13) et N14 (SEQ ID n°14) est effectuée sur cet ADN.
- Primer N13: 5'-CAGATCTGCATGCCTAAAGTTTACAGTTACC-3'(SEQ ID n°13), les bases 1 à 30 sont homologues aux 30 nucléotides de l'extrémité 5'- de la phase de lecture du gène YCYB5 de levure.
- Primer N14:
   5'-CGGATTCTGCAGTTATTCGTTCAACAAATAATAAGCAACACC-3'(SEQ ID n°14), les bases 1 à 42 sont complémentaires des nucléotides 321 à 363 du brin codant pour le b5 de levure.

Parmi six clones analysés, trois clones ont une bande amplifiée à 363 pb (la phase codante du gène du cytochrome b5 de levure), les trois autres clones ont une bande de 2063 pb qui correspond à la somme de la taille du gène HIS3 (1700 pb) et la taille de la phase ouverte du b5 de levure (363 pb). Ces clones sont sous-clonés sur milieu solide W0ABIF pour tester leur auxotrophie vis-à-vis de l'histidine. Tous les clones poussent en absence d'histidine alors que 50 % d'entre eux (ceux qui ont la bande PCR amplifiée à 363 pb) sont sensés être auxotrophes à l'histidine. Dans la souche d'origine W(N), le gène *his3*-11 est muté. Lors de la construction de la souche W(ΔB) (Truan et al., 1993), la disruption du gène YCYB5 de levure par intégration du gène HIS3 dans la phase codante du YCYB5 s'est probablement accompagnée par une deuxième intégration du gène HIS3 à un autre locus. Ceci aurait conduit à la construction d'une souche (W(ΔB)) qui contient deux copies fonctionnelles du gène HIS3. La ségrégation du gène HIS3 ne serait plus du type 2/2. Ceci pourrait expliquer le résultat obtenu. Deux de ces clones sont retenus pour la suite (B1 et B2).

### Exemple n°3 : Construction de la souche W(hR, hb5):

Les 4 clones SP1, SP2, C1 et C2 sont cultivés en milieu complet liquide YPGalA et transformés par le fragment Pvu*II* de 2022pb du vecteur pAB3 (Fig 12). Les transformants sont étalés sur milieu YPGA. La sélection des intégrants se fait sur la capacité du cytochrome b5 humain à sauver de la létalité la souche W(hR, ΔB) sur milieu glucosé. Les clones qui ont poussé sont regroupés par lots. L'ADN génomique de chaque lot est préparé. Une PCR utilisant les primers N5 (SEQ ID n°5) et N15 (SEQ ID n°15) est effectuée (mêmes conditions que les PCR 1 et 2 du paragraphe 8). Les lots qui donnent une bande amplifiée à 758 pb, sont analysés individuellement par PCR. Un clone ayant intégré la cassette d'expression du cytochrome b5 humain au locus YCYB5, W(hR, hb5), est obtenu à partir du clone initial W(hR, ΔB) Sp1.
- Primer N15: 5'-CCgaattcTGATCAGTCCTCTGCCATGTATAGG-3'(SEQ ID n°15), bases 1 à 2: GC clamp, les bases 3 à 8 correspondent au site *Eco*RI (lettres minuscules), les bases 9 à 14 correspondent au site *Bcl*I*,* les bases 12 à 14 correspondent au codon stop et les bases 15 à 33 sont complémentaires des nucléotides 386 à 405 de la phase ouverte de lecture du gène du cytochrome b5 humain.

### Exemple n°4 : Construction de la souche W(GhR, hb5):

Les clones B1 et B2 sont cultivés en milieu complet YPGA liquide et transformés par le fragment de 2022 pb *Pvu*II du vecteur pAB3, ainsi que par le vecteur pPL100 comme marqueur de cotransformation. Les transformants sont sélectionnés sur milieu W0BDIF solide. Les clones qui poussent sont ensuite regroupés par lots et analysés par PCR en utilisant les primers N3 (SEQ ID n°3) et N13 (SEQ ID n°13). De la même façon qu'au paragraphe précédent, les clones individuels sont vérifiés par PCR.

### Exemple n°5 : Construction de la souche W(hR, Lhb5):

Le vecteur d'intégration pAP1 est coupé par *Xba*I. Les 4 clones SP1, SP2, C1 et C2 sont transformés par ce fragment linéarisé. Les transformants sont étalés sur milieu YPGA. Par recombinaison homologue dans la levure, la cassette d'expression du cytochrome b5 humain sous la dépendance du promoteur et du terminateur du gène PGK, s'intègre au site intergénique leu2/SPL1 (Fig. 14). Cette recombinaison permet en même temps de remplacer le gène *leu*2-3 inactif dans le génome par LEU2 sauvage porté par le vecteur pAP1. Une première sélection des transformants se fait sur la restitution du gène LEU2, c'est à dire la prototrophie des transformants vis-à-vis de la leucine (milieu de sélection W0AI). Une deuxième sélection se fait par PCR sur l'ADN génomique des transformants en utilisant les primers N9 (SEQ ID n°9) et N16 (SEQ ID n°16), et les conditions PCR décrites au paragrape 9. Les clones qui présentent une bande PCR à 1495 pb ont intégré la cassette d'expression du hb5.
- Primer N16: 5'-GCCCAGATCTATGGCAGAGCAGTCGGACG-3'(SEQ ID n°16), comporte de la base 1 à 4 une séquence GC clamp, de la base 5 à 10 un site *Bgl*II et de la base 11 à 29 une séquence homologue aux nucléotides 1 à 19 (à partir de l'ATG) de la phase ouverte du gène du cytochrome b5 humain.

### Exemple n°6 : Construction de la souche W(GhR, Lhb5):

La construction de cette souche est faite de la même façon que la souche W(hR, Lhb5) mais à partir de W(GhR, ΔB). La sélection des transformants est faite directement sur milieu W0ABI puis par analyse PCR sur l'ADN génomique.

### Exemple n°7 : Construction de la souche W(R, Lhb5, Yb5):

La souche W(R) est cultivée en milieu complet YGPA liquide et transformée par le vecteur pAP1 linéarisé par *Xba*1. Une première sélection des transformants se fait sur la restitution du gène LEU2, c'est à dire la prototrophie des transformants vis-à-vis de la leucine (milieu de sélection W0AI). Une deuxième sélection se fait par PCR sur l'ADN génomique des transformants en utilisant les primers N9 (SEQ ID n°9) et N16 (SEQ ID n°16), et les conditions PCR décrites au paragraphe 9 (matériel et méthodes). Les clones qui présentent une bande PCR à 1495 pb ont intégré la cassette d'expression du hb5. Cette souche surexprime la réductase de levure en présence de galactose et exprime le cytochrome b5 humain sous le contrôle du promoteur PGK. L'expression du b5 endogène est inchangé par rapport à la souche normale.

### Exemple n°8 : Construction de la souche W(hR, Lhb5, Yb5):

La construction est identique à celle de la souche W(R, Lhb5, Yb5) mais on remplace la souche W(R) par la souche W(hR).

### Exemple n°9 : Niveau d'expression des cytochromes P450 dans les souches de levure selon l'invention.

Les niveaux d'expression de deux cytochromes P450 dans les différentes souches sont indiqués dans les tableaux II et III. De manière surprenante, le niveau d'expression des cytochromes P450 est à conditions de culture équivalente significativement augmenté dans les souches humanisées. On note que l'activité par mg de protéine est accrue proportionnellement à la fois au niveau d'expression et à la valeur du turnover

**Tableau II :**

| expression du cytochrome P450 1A1 humain dans les microsomes de levure humanisée. | | | |
|---|---|---|---|
| Souches | P450 (µM) | Protéine (mg/ml)^{a} | P450 (pmole/mg)^{b} |
| W(N) | 1,6 | 20 | 80 |
| W(R) | 4,5 | 30 | 150 |
| W(R, Lhb5,Yb5) | 4,1 | 31 | 130 |
| W(hR) | 4,4 | 28 | 155 |
| W(hR, Lhb5) | 5,6 | 22 | 260 |
| W(GhR,ΔB) | 6,6 | 26 | 250 |
| W(GhR, Lhb5) | 10,1 | 29,5 | 342 |

| | | | |
|---|---|---|---|
| ^{a} concentration en cytochrome P450 (détermination spectrale) de la suspension de microsomes. | | | |
| ^{b}concentration en protéines totales de la solution de microsomes. | | | |

**Tableau III :**

| expression du cytochrome P450 3A4 humain dans les microsomes de levure humanisée | | | |
|---|---|---|---|
| Souches | P450 (µM)^{a} | Protéine (mg/ml)^{b} | P450 (pmole/mg) |
| W(N) | | | 80 |
| W(R) | 10,4 | 32 | 320 |
| W(R, Lhb5,Yb5) | 9,1 | 30 | 300 |
| W(hR) | 10,5 | 34 | 310 |
| W(hR, Lhb5) | 4 | 23 | 180 |
| W(GhR,ΔB) | 7,4 | 29 | 250 |
| W(GhR, Lhb5) | 9 | 22 | 410 |

| | | | |
|---|---|---|---|
| ^{a} concentration en cytochrome P450 (détermination spectrale) de la suspension de microsomes. | | | |
| ^{b}concentration en protéines totales de la solution de microsomes. | | | |

### Exemple n°10 : Effet de la réductase et du cytochrome b5 sur les caractéristiques enzymatiques des cytochromes P450 1A1 et 3A4 produit chez la levure.

Les résultats obtenus indiquent que l'efficacité catalytique du cytochrome P450 1A1 est optimale dans la souche haploïde W(R, LHb5) qui surproduit la réductase de levure et produit le cytochrome b5 humain. L'adjonction de cytochrome b5 de lapin produit néanmoins une légère augmentation supplémentaire de l'activité qui atteint un turnover de 27 pmole de métabolite/ pmole de cytochrome P450 par min.

A l'inverse des résultats obtenus avec le cytochrome P450 1A1, l'efficacité catalytique du cytochrome P450 3A4 est préférentiellement accrue dans les souches de levures exprimant la réductase humaine. La souche W(GhR, hb5) montre une efficacité catalytique optimale. Quelle que soit la nature et le niveau de la réductase, la présence de l'ADNc codant pour le cytochrome b5 dans le génome des souches se traduit par une augmentation de l'efficacité catalytique (d'un facteur 2 à 20). La délétion du gène endogène du cytochrome b5 apparaît comme un facteur fortement favorable particulièrement en présence de b5 humain exprimé. L'expression de la réductase humaine, la disruption du cytochrome b5 endogène et l'expression du b5 humain dans une même souche de levure haploïde constitue un ensemble particulièrement favorable à l'expression de certains cytochrome P450 humains, en particulier le cytochrome P450 3A4.

### Exemple 11 : Construction de plasmides permettant la co-expression d'un cytochrome P450 et du cytochrome b5 microsomal humain.

Cet exemple décrit la construction de plasmides utilisables pour optimiser l'activité P450 des souches de levure selon l'invention. Ces plasmides permettent l'expression simultanée d'un cytochrome P450 et du cytochrome b5 microsomal humain. Tous les plasmides de la série comportent l'ensemble original des caractères communs suivants:
(i) La cassette d'expression du P450 est préférentiellement sous le contrôle transcriptionnel du promoteur inductible GAL10-CYC1.
(ii) La cassette d'expression du cytochrome b5 est préférentiellement sous le contrôle transcriptionnel d'un des trois promoteurs suivants: GAL10-CYC1, GAPDH ou PGK.
(iii) Deux marqueurs de sélection sont présents dont l'un est préférentiellement le gène ADE2 de levure.
(iv) Les deux cassettes d'expression sont séparées sur le plasmide d'un côté par l'origine de réplication levure, de l'autre coté par le marqueur ADE2. Dans le cas où deux promoteurs identiques de type GAL10-CYC1 sont utilisés pour l'expression du b5 et du P450, ces promoteurs ont des orientations inversées sur le plasmide (par rapport à une rotation du plasmide). Deux terminateurs de transcription différents sont utilisés, préférentiellement ceux des gènes PGK et de la P450 réductase de levure. L'ensemble de ces propriétés a pour objet l'obtention de plasmides stables en particulier par rapport aux phénomènes de recombinaison homologue.
Les plasmides de la série diffèrent par la nature du promoteur utilisé pour l'expression du cytochrome b5. Cette propriété permet de moduler les niveaux relatifs d'expression et de les optimiser pour différentes conditions de culture ou applications.

### Exemple 11.1. Construction du plasmide pAP4 permettant l'expression d'un P450 sous le contrôle du promoteur GAL10-CYC1 et du b5 humain sous promoteur PGK.

Le vecteur, décrit dans la figure 15, pYeDP1/10/Hb5, contient la POL (phase ouverte de lecture) du cytochrome b5 humain bordée immédiatement en amont du codon d'initiation par un site BglII et immédiatement en aval du codon STOP par un site EcoRI. La cassette BglII-EcoRI étant incluse dans le vecteur d'expression pYeDP1/10 (Cullin et Pompon, Gene. 65 (1988) 203-217). Ce vecteur est digéré par BamHI et HindIII. Le fragment de 2310 pb qui contient les séquences du promoteur et du terminateur du gène PGK et la séquence codant pour le cytochrome b5 humain est récupéré puis traité par le fragment de Klenow de l'ADN polymérase afin de rendre les extrémités bouts francs. Le vecteur pYeDP60 est digéré par l'enzyme de restriction EcoRV et le fragment de 5819 pb récupéré. Celui-ci contient les origines de réplication pour la levure et E. Coli. Ce fragment est recircularisé sur lui-même et donne le vecteur pAP2. Ce vecteur est digéré par PvuII et le fragment de 2310 pb issu du vecteur pYeDP1/10/Hb5 y est inséré dans l'orientation donnant le vecteur pAP3 dont la casette d'expression est conforme à la figure 17. Ce vecteur est digéré BgII. Le fragment de 7012 pb est récupéré. Le vecteur pYeDP60 est linéarisé par PvuII et la bande qui correspond au plasmide linéarisé est purifiée. Une culture de levure issue d'un clone de la souche W(N) est cotransformée par le plasmide linéaire et le fragment de 7012 pb et des clones prototrophes pour l'uracile et l'adénine sont sélectionnés. Par recombinaison homologue dans la levure, la cassette d'expression du cytochrome b5 sous le contrôle du promoteur et du terminateur du gène PGK se substitue au site PvuII du vecteur pYeDP60 et donne le vecteur final pAP4 (figures 16 et 17). L'ADN plasmidique correspondant au vecteur pAP4 est récupéré de la levure et utilisé pour transformer E. coli (Amp^{r}). Après sélection, amplification et contrôle de la structure du plasmide par restriction, la POL du cytochrome P450 d'intérêt est insérée dans le vecteur pAP4 dans la zone de clonage multiple située entre le promoteur GAL10-CYC1 et le terminateur du gène PGK. Le plasmide résultant est utilisé pour transformer, par sélection de clones prototrophes pour l'adénine et l'uracile, la souche de levure réceptrice choisie préférentiellement parmi celles décrites dans le présent brevet.

### Exemple 11.2. Construction du plasmide pVD2 permettant l'expression d'un P450 sous le contrôle du promoteur GAL10-CYC1 et du b5 humain sous promoteur GAPDH.

Le vecteur pYeDP1/10/Hb5 est digéré par BglII et EcoRI pour sortir la bande codant pour le cytochrome b5 humain. La bande de 417 pb correspondant à la POL du b5 est purifiée puis clonée dans pAB2 digéré par BamHI et EcoRI. Dans le vecteur obtenu, le site EcoRI est alors détruit par digestion par EcoRI puis remplissage du site à l'aide du fragment de klenow de la DNA polymérase puis recircularisation par la ligase .Ce plasmide de 4993 pb, est appelé pVD1 (figures 16 et 18).

La cassette d'expression de pVD1 est ensuite amplifiée par PCR en utilisant les primers N17 (SEQ ID n° 17) et N18 (SEQ ID n° 18) comme amorces. La bande amplifiée est de 1753 pb. Les primers sont conçus pour ajouter aux deux extrémités de la cassette d'expression des pieds de recombinaisons homologues à la région située autour du site unique PvuII de pYeDP60. Par recombinaison homologue (voir exemple 11.1. et Figure 17) entre pYeDP60 linéarisé au site PvuII et déphosphorylé par la phosphatase alcaline, et la cassette d'expression amplifiée par PCR, le vecteur pVD2 conforme aux figures 16 et 19 est alors obtenu. La structure du vecteur est vérifiée après navette chez la levure (cotransformation) puis E. coli (sélection et amplification) par digestion par PstI et HindIII. Le plasmide souhaité présentant une bande de digestion à 10994 pb est appelé pVD2 (Figure 19). Les différentes jonctions de la cassette d'expression sont vérifiées par séquençage à l'aide des primers N19 (SEQ ID n°19) et N20 (SEQ ID n°20).

### Exemple 1.3. Construction du plasmide pVD3 permettant l'expression d'un P450 sous le contrôle du promoteur GAL10-CYC1 et du b5 humain sous promoteur GAL10-CYC1.

De même que dans la construction précédente, la POL du cytochrome b5 humain est obtenue à partir de pYeDP1/10/hb5 sous forme de fragment BglII-EcoRI. Cette POL est introduite par ligation entre les sites BamHI et EcoRI du plasmide pYeDP110, décrit figure 15, qui contient un bloc de séquence constitué (i) de séquences situées immédiatement en amont de la région promotrice du gène de la P450 réductase de levure, (ii) du gène URA3, (iii) du promoteur GAL10-CYC1, (iv) du terminateur de transcription du gène de la P450 réductase de levure. Comme précédemment le site EcoRI du plasmide résultant de la ligation est détruit par coupure-remplissage-ligation pour donner un plasmide de 5551 pb appelé pVD3 (Figure 20). La cassette d'expression du cytochrome b5 humain sous le promoteur GAL10-CYC1 est alors amplifiée par PCR grâce aux amorces N17 et N18 de manière similaire à l'exemple 12. La bande amplifiée de 2365 pb est purifiée puis introduite comme précédemment par recombinaison homologue en lieu du site PvuII de pYeDP60 (idem que la figure 19 en substituant le promoteur GAPDH par GAL10-CYC1). La nature des séquences des pieds de recombinaison introduits par PCR impose une orientation inverse des deux cassettes GAL10-CYC1 présentent sur le plasmide recombiné comme indiqué dans la description de la structure générale des plasmides de l'invention. Le plasmide de 11595 pb, appelé pVD4 et conforme à la figure 16 est alors sélectionné après navette levure-Ecoli La jonction entre le promoteur GAL10-CYC1 et le gène du cytochrome b5 est vérifiée par séquençage grâce à l'amorce N21 (SEQ ID n°21).

**Nomenclature des souches obtenues:**
W(GhR, ΔB): *MAT*a*, leu2-*3,112, *ade2*-1, *trp1*-1, *ura3*-1.
W(hR, ΔB): *MAT*a, *leu2*-3,112, *ade2*-1, *trp1-*1.
W(hR, hb5): *MAT*a*, leu2*-3,112, *ade2*-1*, trp1*-1*.*
W(GhR, hb5): *MAT*a*, leu2*-3,112, *ade2*-1, *trp1*-1*, ura3*-1.
W(hR, Lhb5): *MAT*a, *ade2*-1, *trp1*-1.
W(GhR, Lhb5): *MAT*a, *ade2*-1, *trp1*-1, *ura3*-1.
W(R, Lhb5, Yb5)
W(hR, Lhb5, Yb5)

### Séquences des primers utilisés :

Les 42 premières bases sont homologues à la région comprise entre les bases 4069 et 4113 de pYeDP60. En majuscules, bases 43-69, les nucléotides du primer sont homologues à la région de pUB81 comprise entre les bases 5772 et 5799 qui correspondent à une région du gène de URA3.

Les 43 premières bases sont homologues au brin complémentaire de la région de pYeDP60 comprise entre les bases 4114 et 4157. Les bases 44 à 71 sont homologues à la région de pUB81 comprise entre les bases 2507 et 2538.

Ce primer est homologue de la région entre 4102 à 4125 de pVD2 correspondant à la fin de la phase ouverte de lecture du gène URA3 voisin de la cassette d'intégration du cytochrome b5 humain sous le promoteur GAPDH.

Cette amorce pour le séquençage est homologue de la partie de pVD2 comprise entre les bases 4835 et 4860 correspondant à la fin du promoteur GAPDH.

Cette amorce pour le séquençage est homologue à la région du promoteur GAL10-CYC1 située à -120 pb de la jonction entre le promoteur GAL10-CYC1 et la POL du cytochrome b5 humain.

### REFERENCES:

- Baim, S. B. & Scherman, F. (1988) mRNA structures influencing translation in the yeast S. *cerevisae, Mol. Cell. Biol. 8,* 1591-1601.
- Barnes, H. J. Arlotto, M. P. & Waterman, M. R. (1991) Expression and enzymatic activity of recombinant cytochrome P450 17a-hydroxylase in *Escherichia coli Proc. Natl. Acad. Sci. USA 88*, 5597-5601.
- Bellamine, A. Gautier, J. C. Urban, P. & Pompon, D. (1994) Chimeras of the human cytochrome P450 1A family produced in yeast: Accumulation in microsomal membranes, enzyme kinetics and stability, *Eur. J. Biochem. 225,* 1005-1013.
- Bonneaud, N. Ozier-Kalogeropoulos, O. Li, G. Labrousse, M. Minvielle-Sebastia, L. & Lacroute, F. (1991) A family of low and high copy replicative, integrative and single strand *S.cerevisiae*/*E. coli* shuttle vectors, *Yeast 7,* 609-615.
- Cullin, C. & Pompon, D. (1988) Synthesis of functional mouse cytochrome P450 P1 and chimeric cytochrome P450 P3-1 in the yeast *Saccharomyces cerevisiae, Gene* (*Amst.*) *65,* 203-217.
- Doehmer, J. & Greim, H. Cytochromes P450 in genetically engineered cell cultures: the gene technological approch in *Cytochrome P450,* Handbook of experimental pharmacology, 105; Eds Schenkman, J. B. & Greim, H. Springer-Verlag, Berlin, Heidelberg, New York (1992), 3-13.
- Guengerich, F. P. (1988) Roles of cytochrome P450 enzymes in chemical carcinogenesis and cancer chemotherapy, *Cancer Res. 48,*2946-2954.
- Oeda, K. Sakaki, T. & Ohkawa, H. (1985) Expression of rat liver cytochrome P-450MC cDNA in *Saccharomyces cerevisiae, DNA 4,*203-210.
- Pompon, D. (1988) cDNA cloning and functional expression in yeast *Saccharomyces cerevisiae* of b-naphtoflavone-induced rabbit liver cytochromes P-450 LM4 and LM6, *Eur. J. Biochem. 177,* 285-293.
- Stotz, A. & Linder, P. (1990) The ADE2 gene from Saccharomyces cerevisiae: séquence and new vectors, *Gene (Amst.) 95,* 91-98.
- Truan, G. Cullin, C. Reisdorf, P. Urban, P. & Pompon, D. (1993) Enhanced *in vivo* monooxygenase activities of mammalian cytochrome P450s in engineered yeast cells producing high levels of NADPH-cytochrome P450 réductase and human cytochrome b5, *Gene (Amst) 125,* 49-55.
- Truan, G. Epinat, J. C. Rougeulle, C. Cullin, C. & Pompon, D. (1994) Cloning and charactherization of yeast cytochrome b5-encoding gene wich supresses ketokonazole hypersensivity in a NADPH-P-450 réductase-deficient strain, *Gene (Amst.) 142*, 123-127.
- Urban, P. Cullin, C. & Pompon, D. (1990) Maximizing the expression of mammalian cytochrome P-450 monooxygenase activities in yeast cells, *Biochimie (Paris) 72,* 463-472.
- Urban, P. Truan, G. Gautier, J. C. & Pompon D (1993) Xenobiotic metabolism in humanized yeast: engenieered yeast cells producing human NADPH-cytochrome P450 réductase, cytochrome b5, epoxide hydrolase and cytochrome P450s, *Biochem Soc Transact 21,*1028-1033.
- Zuber, M. X. Simpson, E. R. & Waterman, M. R. (1986) Expression of bovine 17a-hydroxylase cytochrome P-450s cDNA in nonsteroidogenic (COS1) cells, *Science 234,* 1258-1261.
- Brevet n° WO 93/02200: Souches de levure avec intégration stable de gènes hétérologues.
- Brevet n° WO 94/01564: Souche de levure permettant la co-expression d'une activité mono-oxygénase de cytochrome P450 de plante et d'une NADPH-cytochrome P450-réductaseendogène ou hétérologue et son utilisation à des fins de bioconversion.

### LISTE DE SEQUENCES

(1) INFORMATIONS GENERALES:
   (i) DEPOSANTS:
      (A) NOM: RHONE POULENC RORER S.A.
      (B) RUE: 20, AVENUE RAYMOND ARON
      (C) VILLE: ANTONY
      (E) PAYS: FRANCE
      (F) CODE POSTAL: 92165
      (G) TELEPHONE: (1) 40.91.69.22
      (H) TELECOPIE: (1) 40.91.72.91

      (A) NOM: C.N.R.S
      (B) RUE: 3, Rue Michel Ange
      (C) VILLE: PARIS
      (E) PAYS: FRANCE
      (F) CODE POSTAL: 75016
      (G) TELEPHONE: (1) 44 96 40 00
      (H) TELECOPIE: (1) 44 96 50 00
   (ii) TITRE DE L' INVENTION: SOUCHES DE LEVURES EXPRIMANT DES GENES HUMAINS LEUR MODE DE PREPARATION AINSI QUE LES SOUCHES INTERMEDIAIRES.
   (iii) NOMBRE DE SEQUENCES: 21
   (iv) FORME DECHIFFRABLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: Patent In Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 27 bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 1:
   GCGGATCCAT GGGAGACAGT CACGTGG 27
(2) INFORMATIONS POUR LA SEQ ID NO: 2:
   (i) CARACTERISTIQUES DE LA SEQUENCE:
      (A) LONGUEUR: 31 bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 2:
   CGGAATTCAG ATCTAGCTCC ACACGTCCAG G 31
(2) INFORMATIONS POUR LA SEQ ID NO: 3:
   (i) CARACTERISTIQUES DE LA SEQUENCE:
      (A) LONGUEUR: 31 bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 3:
   CCAAGCTTGA GTTTATCATT ATCAATACTC G 31
(2) INFORMATIONS POUR LA SEQ ID NO: 4:
   (i) CARACTERISTIQUES DE LA SEQUENCE:
      (A) LONGUEUR: 42 bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 4:
   CGGATCCTAT TTATGTGTGT TTATTCGAAA CTAAGTTCTT GG 42
(2) INFORMATIONS POUR LA SEQ ID NO: 5:
   (i) CARACTERISTIQUES DE LA SEQUENCE:
      (A) LONGUEUR: 31 bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 5:
   GGATCCGAGC GGGTAATAGC CTGGAGTTTC C 31
(2) INFORMATIONS POUR LA SEQ ID NO: 6:
   (i) CARACTERISTIQUES DE LA SEQUENCE:
      (A) LONGUEUR: 48 bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 6:
   CCGACTGCTC TGCCATGATT GTTTGATATT TTATGTTGTA GTTGATTG 48
(2) INFORMATIONS POUR LA SEQ ID NO: 7:
   (i) CARACTERISTIQUES DE LA SEQUENCE:
      (A) LONGUEUR: 50 bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 7:
   CCTATACATG GCAGAGGACT GAATTCTTTT TCTTCCAGAA TAGCCCACAC 50
(2) INFORMATIONS POUR LA SEQ ID NO: 8:
   (i) CARACTERISTIQUES DE LA SEQUENCE:
      (A) LONGUEUR: 31 bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 8:
(2) INFORMATIONS POUR LA SEQ ID NO: 9:
   (i) CARACTERISTIQUES DE LA SEQUENCE:
      (A) LONGUEUR: 25 bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 9:
   TTGAAGGTTC AACATCAATT GATTG 25
(2) INFORMATIONS POUR LA SEQ ID NO: 10:
   (i) CARACTERISTIQUES DE LA SEQUENCE:
      (A) LONGUEUR: 38 bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION.DE LA SEQUENCE: SEQ ID NO: 10:
(2) INFORMATIONS POUR LA SEQ ID NO: 11:
   (i) CARACTERISTIQUES DE LA SEQUENCE:
      (A) LONGUEUR: 34 bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 11:
   CAAGGAGGCG GCCGCCACAC AAAAAGTTAG GTGT 34
(2) INFORMATIONS POUR LA SEQ ID NO: 12:
   (i) CARACTERISTIQUES DE LA SEQUENCE:
      (A) LONGUEUR: 25 bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 12:
( 2) INFORMATIONS POUR LA SEQ ID NO: 13:
   (i) CARACTERISTIQUES DE LA SEQUENCE:
      (A) LONGUEUR: 31 bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 13:
(2) INFORMATIONS POUR LA SEQ ID NO: 14:
   (i) CARACTERISTIQUES DE LA SEQUENCE:
      (A) LONGUEUR: 42 bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 14:
   CGGATTCTGC AGTTATTCGT TCAACAAATA ATAAGCAACA CC 42
(2) INFORMATIONS POUR LA SEQ ID NO: 15:
   (i) CARACTERISTIQUES DE LA SEQUENCE:
      (A) LONGUEUR: 33 bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 15:
   CCGAATTCTG ATCAGTCCTC TGCCATGTAT AGG 33
(2) INFORMATIONS POUR LA SEQ ID NO: 16:
   (i) CARACTERISTIQUES DE LA SEQUENCE:
      (A) LONGUEUR: 29 bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 16:
   GCCCAGATCT ATGGCAGAGC AGTCGGACG 29
(2) INFORMATIONS POUR LA SEQ ID NO: 17:
   (i) CARACTERISTIQUES DE LA SEQUENCE:
      (A) LONGUEUR: 69 bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 17:
   GGCGTTACCC AACTTAATCG CCTTGCACAT CCCCCTTTCG CCGGAACGTG 50
   CTGCTACTCA TCCTAGTCC 69
(2) INFORMATIONS POUR LA SEQ ID NO: 18:
   (i) CARACTERISTIQUES DE LA SEQUENCE:
      (A) LONGUEUR: 70 bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 18:
   GGGAAGGGCG ATCGGTGCGG GCCTCTTCGC TATTACGCCA GCTGGCCGAT 50
   TCATTAATGC AGCTGGGCGG 70
(2) INFORMATIONS POUR LA SEQ ID NO: 19:
   (i) CARACTERISTIQUES DE LA SEQUENCE:
      (A) LONGUEUR: 24 bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 19:
   CCCCCCTTTC GCCAGGGAAC GTGC 24
(2) INFORMATIONS POUR LA SEQ ID NO: 20:
   (i) CARACTERISTIQUES DE LA SEQUENCE:
      (A) LONGUEUR: 24 bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 20:
   CGGTAGGTAT TGATTGTAAT TCTG 24
(2) INFORMATIONS POUR LA SEQ ID NO: 21:
   (i) CARACTERISTIQUES DE LA SEQUENCE:
      (A) LONGUEUR: 21 bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 21:
   GGCATGCATG TGCTCTGTAT G                                            21

## Revendications

1. Souche de levure génétiquement modifiée **caractérisée en ce que** :
1° Les gènes codant pour le cytochrome b5 endogène et pour la cytochrome P450 réductase endogène ont été inactivés,
2° Elle contient un acide nucléique codant pour la NADPH-cytochrome P450-réductase humaine,
3° Elle contient un acide nucléique codant pour le cytochrome b5 humain.

2. Souche selon la revendication 1 **caractérisée en ce que** l'acide nucléique est un ADNc.

3. Souche selon la revendication 2 **caractérisée en ce que** l'un au moins des ADNc humains est sous contrôle d'un promoteur de levure constitutif ou inductible.

4. Souche selon la revendication 3 **caractérisée en ce qu'**il s'agit d'un promoteur constitutif, choisi parmi le promoteur du gène de la glyceraldéhyde phosphodéhydrogénase, le promoteur du gène de la phosphoglycérate kinase et le promoteur endogène du cytochrome b5.

5. Souche selon la revendication 3 **caractérisée en ce que** le promoteur inductible est choisi parmi les promoteurs GAL10 et CYC1-GAL10

6. Souche selon l'une des revendications 1 à 5 **caractérisée en ce que** l'acide nucléique codant pour la NADPH-cytochrome P450-réductase humaine est intégré dans le génome de la levure.

7. Souche selon l'une des revendications 1 à 6 **caractérisée en ce que** l'acide nucléique codant pour le cytochrome b5 humain est intégré dans le génome de la levure.

8. Souche selon l'une des revendications 1 à 7 **caractérisée en ce qu'**elle comprend en outre au moins un acide nucléique codant pour un cytochrome P450 humain.

9. Souche selon la revendication 8 **caractérisée en ce que** l'acide nucléique codant pour le cytochrome P450 humain est intégré sur un plasmide.

10. Souche selon la revendication 9 **caractérisée en ce qu'**elle comprend en outre une copie supplémentaire de l'acide nucléique codant pour le cytochrome b5 humain sur ledit plasmide ou intégré dans le génome.

11. Souche selon l'une des revendications précédentes **caractérisée en ce qu'**elle est haploïde.

12. Souche selon l'une quelconque des revendications 1 à 11 **caractérisée en ce que** la levure est une souche de *Saccharomyces cerevisiae.*

13. Souche selon l'une quelconque des revendications 1 à 7, 11 et 12 **caractérisée en ce qu'**il s'agit d'une souche qui comprend un acide nucléique codant pour le cytochrome b5 humain et un acide nucléique codant pour la NADPH-cytochrome P450-réductase humaine.

14. Souche selon la revendication 13 **caractérisée en ce que** l'acide nucléique codant pour la NADPH-cytochrome P450-réductase humaine y est sous contrôle du promoteur du gène de la glyceraldéhyde phosphodéhydrogénase de levure.

15. Souche selon la revendication 13 **caractérisée en ce que** l'acide nucléique codant pour le cytochrome b5 humain y est sous contrôle du promoteur du gène de la phosphoglycérate kinase de levure.

16. Souche selon l'une des revendications 13, 14 ou 15 **caractérisée en ce que** l'acide nucléique codant pour le cytochrome b5 humain y est sous contrôle du promoteur du gène de la phosphoglycérate kinase de levure et l'acide nucléique codant pour la NADPH-cytochrome P450-réductase humaine y est sous contrôle du promoteur du gène de la glyceraldéhyde phosphodéhydrogénase de levure.

17. Souche selon les revendications 13 à 16 **caractérisée en ce qu'**elle comprend en outre au moins un acide nucléique codant pour un cytochrome P450 humain.

18. Souche selon la revendication 17 **caractérisée en ce que** l'acide nucléique codant pour le cytochrome P450 humain est intégré sur un plasmide.

19. Souche de levure génétiquement modifiée comprenant un acide nucléique codant pour la NADPH-cytochrome P450-réductase humaine et dont les gènes codant pour le cytochrome b5 de levure et la NADPH-cytochrome P450-réductase de levure ont été inactivés.

20. Souche selon la revendication 19 **caractérisée en ce que** l'acide nucléique codant pour la NADPH-cytochrome P450-réductase humaine est sous contrôle du promoteur du gène de la glyceraldéhyde phosphodéhydrogénase de levure.

21. Procédé d'obtention de souches selon la revendication 13 ou la revendication 15 **caractérisé en ce qu'**il met en oeuvre une souche de levure comprenant un acide nucléique codant pour la NADPH-cytochrome P450-réductase humaine sous contrôle du promoteur GAL10-CYC1 et dont les gènes codant pour le cytochrome b5 de levure et la NADPH-cytochrome P450-réductase de levure ont été inactivés.

22. Procédé d'obtention de souches selon la revendication 14 ou 16 **caractérisé en ce qu'**il met en oeuvre une souche de levure comprenant un acide nucléique codant pour la NADPH-cytochrome P450-réductase humaine sous contrôle du promoteur de la glycéraldehyde phosphodehydrogénase et dont les gènes codant pour le cytochrome b5 de levure et la NADPH-cytochrome P450-réductase de levure ont été inactivés.

23. Procédé d'évaluation de la toxicité d'un composé **caractérisé en ce que** :
- le dit composé est mis en présence d'une levure selon l'une des revendications 1 à 18 ou d'une préparation enzymatique dérivée d'une telle levure et
- on analyse les métabolites produits quant à leur toxicité.

24. Méthode de détermination in vitro des métabolites humain d'un composé chimique **caractérisée en ce que** :
- le dit composé est mis en présence d'une levure selon l'une des revendications 1 à 18 ou d'une préparation enzymatique dérivée d'une telle levure et
- on analyse les métabolites produits.

25. Plasmide **caractérisé en ce qu'**il permet l'expression simultanée dans la levure d'un cytochrome P450, de préférence humain, et du cytochrome b5 microsomal humain.

26. Plasmide selon la revendication 25 **caractérisé en ce que**:
(i) la cassette d'expression du P450 comprend préférentiellement le promoteur GAL10-CYC1,
(ii) la cassette d'expression du b5 comprend préférentiellement l'un des promoteurs GAL10-CYC1, PGK ou GAPDH, et,
(iii) lorsque le même promoteur est utilisé deux fois, les deux copies sont présentes dans des orientations inversées sur le plasmide.

27. Plasmide selon la revendication 26 **caractérisé en ce que** les deux cassettes d'expression sont séparées sur le plasmide d'un côté par l'origine de réplication levure, de l'autre côté par au moins un marqueur de sélection levure.

28. Souche de levure transformée par un plasmide selon l'une des revendications 25 à 27.

29. Souche de levure selon la revendication 28 dépourvue du gène endogène du cytochrome b5 microsomal.

## Patentansprüche

1. Genetisch veränderter Hefestamm, **dadurch gekennzeichnet, dass**:
1. die Gene, die für endogenes Cytochrom b5 und für endogene Cytochrom P450-Reduktase kodieren, inaktiviert wurden,
2. er eine Nukleinsäure enthält, die für humane NADPH-Cytochrom P450-Reduktase kodiert,
3. er eine Nukleinsäure enthält, die für humanes Cytochrom b5 kodiert.

2. Stamm gemäß Anspruch 1, **dadurch gekennzeichnet, dass** die Nukleinsäure eine cDNA ist.

3. Stamm gemäß Anspruch 2, **dadurch gekennzeichnet, dass** wenigstens eine der humanen cDNA unter Kontrolle eines konstitutiven oder induzierbaren Hefepromotors steht.

4. Stamm gemäß Anspruch 3, **dadurch gekennzeichnet, dass** es sich um einen konstitutiven Promotor handelt, der ausgewählt ist unter dem Promotor des Gens der Glyceraldehyd-Phosphat-Dehydrogenase, dem Promotor des Gens der Phosphoglycerat-Kinase und dem endogenen Promotor von Cytochrom b5.

5. Stamm gemäß Anspruch 3, **dadurch gekennzeichnet, dass** der induzierbare Promotor ausgewählt ist unter den GAL10- und CYC1-GAL10-Promotoren.

6. Stamm gemäß einem der Ansprüche 1 bis 5, **dadurch gekennzeichnet, dass** die Nukleinsäure, die für humane NADPH-Cytochrom P450-Reduktase kodiert, in das Genom der Hefe integriert ist.

7. Stamm gemäß einem der Ansprüche 1 bis 6, **dadurch gekennzeichnet, dass** die Nukleinsäure, die für humanes Cytochrom b5 kodiert, in das Genom der Hefe integriert ist.

8. Stamm gemäß einem der Ansprüche 1 bis 7, **dadurch gekennzeichnet, dass** er außerdem wenigstens eine Nukleinsäure umfasst, die für ein humanes Cytochrom P450 kodiert.

9. Stamm gemäß Anspruch 8, **dadurch gekennzeichnet, dass** die Nukleinsäure, die für humanes Cytochrom P450 kodiert, auf einem Plasmid integriert ist.

10. Stamm gemäß Anspruch 9, **dadurch gekennzeichnet, dass** er außerdem eine zusätzliche Kopie der Nukleinsäure, die für humanes Cytochrom b5 kodiert, auf besagtem Plasmid oder in das Genom integriert umfasst.

11. Stamm gemäß einem der vorhergehenden Ansprüche, **dadurch gekennzeichnet, dass** er haploid ist.

12. Stamm gemäß einem der Ansprüche 1 bis 11, **dadurch gekennzeichnet, dass** die Hefe ein Stamm von *Saccharomyces cerevisiae* ist.

13. Stamm gemäß einem der Ansprüche 1 bis 7, 11 und 12, **dadurch gekennzeichnet, dass** es sich um einen Stamm handelt, der eine Nukleinsäure, die für humanes Cytochrom b5 kodiert, und eine Nukleinsäure, die für humane NADPH-Cytochrom P450-Reduktase kodiert, umfasst.

14. Stamm gemäß Anspruch 13, **dadurch gekennzeichnet, dass** die Nukleinsäure, die für humane NADPH-Cytochrom P450-Reduktase kodiert, dort unter Kontrolle des Promotors des Gens der Hefe-Glyceraldehyd-Phosphat-Dehydrogenase steht.

15. Stamm gemäß Anspruch 13, **dadurch gekennzeichnet, dass** die Nukleinsäure, die für humanes Cytochrom b5 kodiert, dort unter Kontrolle des Promotors des Gens der Hefe-Phosphoglycerat-Kinase steht.

16. Stamm gemäß einem der Ansprüche 13, 14 oder 15, **dadurch gekennzeichnet, dass** die Nukleinsäure, die für humanes Cytochrom b5 kodiert, dort unter Kontrolle des Promotors des Gens der Hefe-Phosphoglycerat-Kinase steht, und die Nukleinsäure, die für humane NADPH-Cytochrom P450-Reduktase kodiert, dort unter Kontrolle des Promotors des Gens der Hefe-Glyceraldehyd-Phosphat-Dehydrogenase steht.

17. Stamm gemäß den Ansprüchen 13 bis 16, **dadurch gekennzeichnet, dass** er außerdem wenigstens eine Nukleinsäure umfasst, die für ein humanes Cytochrom P450 kodiert.

18. Stamm gemäß Anspruch 17, **dadurch gekennzeichnet, dass** die Nukleinsäure, die für humanes Cytochrom P450 kodiert, auf einem Plasmid integriert ist.

19. Genetisch veränderter Hefestamm, der eine Nukleinsäure umfasst, die für humane NADPH-Cytochrom P450-Reduktase kodiert, und dessen Gene, die für Hefe-Cytochrom b5 und Hefe-NADPH-Cytochrom P450-Reduktase kodieren, inaktiviert wurden.

20. Stamm gemäß Anspruch 19, **dadurch gekennzeichnet, dass** die Nukleinsäure, die für humane NADPH-Cytochrom P450-Reduktase kodiert, unter Kontrolle des Promotors des Gens der Hefe-Glyceraldehyd-Phosphat-Dehydrogenase steht.

21. Verfahren zum Erhalt von Stämmen gemäß Anspruch 13 oder Anspruch 15, **dadurch gekennzeichnet, dass** es einen Hefestamm einsetzt, der eine Nukleinsäure umfasst, die unter Kontrolle des GAL10-CYC1-Promotors für humane NADPH-Cytochrom P450-Reduktase kodiert, und dessen Gene, die für Hefe-Cytochrom b5 und Hefe-NADPH-Cytochrom P450-Reduktase kodieren, inaktiviert wurden.

22. Verfahren zum Erhalt von Stämmen gemäß Anspruch 14 oder 16, **dadurch gekennzeichnet, dass** es einen Hefestamm einsetzt, der eine Nukleinsäure umfasst, die unter Kontrolle des Promotors der Glyceraldehyd-Phosphat-Dehydrogenase für humane NADPH-Cytochrom P450-Reduktase kodiert, und dessen Gene, die für Hefe-Cytochrom b5 und Hefe-NADPH-Cytochrom P450-Reduktase kodieren, inaktiviert wurden.

23. Verfahren zur Ermittlung der Toxizität einer Verbindung, **dadurch gekennzeichnet, dass**:
- besagte Verbindung mit einer Hefe gemäß einem der Ansprüche 1 bis 18 oder einer Enzymzubereitung, die von einer solchen Hefe abgeleitet ist, zusammengebracht wird und
- man die erzeugten Metaboliten hinsichtlich ihrer Toxizität analysiert.

24. Verfahren zur in vitro-Bestimmung der humanen Metaboliten einer chemischen Verbindung, **dadurch gekennzeichnet, dass**:
- besagte Verbindung mit einer Hefe gemäß einem der Ansprüche 1 bis 18 oder einer Enzymzubereitung, die von einer solchen Hefe abgeleitet ist, zusammengebracht wird und
- man die erzeugten Metaboliten analysiert.

25. Plasmid, **dadurch gekennzeichnet, dass** es die gleichzeitige Expression in der Hefe eines vorzugsweise humanen Cytochroms P450 und von humanem mikrosomalem Cytochrom b5 ermöglicht.

26. Plasmid gemäß Anspruch 25, **dadurch gekennzeichnet, dass**:
(i) die P450-Expressionskassette bevorzugt den GAL10-CYC1-Promotor umfasst,
(ii) die b5-Expressionskassette bevorzugt einen der GAL10-CYC1-, PGK- oder GAPDH-Promotoren umfasst und
(iii) wenn der gleiche Promotor zweimal verwendet wird, die beiden Kopien auf dem Plasmid in umgekehrten Orientierungen vorhanden sind.

27. Plasmid gemäß Anspruch 26, **dadurch gekennzeichnet, dass** die beiden Expressionskassetten auf dem Plasmid auf der einen Seite durch den Hefereplikationsursprung, auf der anderen Seite durch wenigstens einen Hefeselektionsmarker getrennt sind.

28. Hefestamm, der mit einem Plasmid gemäß einem der Ansprüche 25 bis 27 transformiert ist.

29. Hefestamm gemäß Anspruch 28, der frei von endogenem Gen für mikrosomales Cytochrom b5 ist.

## Claims

1. Genetically modified yeast strain, **characterized in that**:
Firstly, the genes encoding the endogenous cytochrome b5 and the endogenous cytochrome P450 reductase have been inactivated,
Secondly, the strain comprises a nucleic acid which encodes human NADPH-cytochrome P450 reductase,
Thirdly, the strain comprises a nucleic acid which encodes human cytochrome b5.

2. Strain according to Claim 1, **characterized in that** the nucleic acid is a cDNA.

3. Strain according to Claim 2, **characterized in that** at least one of the human cDNAs is under the control of a constitutive or inducible yeast promoter.

4. Strain according to Claim 3, **characterized in that** the promoter is a constitutive promoter which is selected from the promoter of the gene for glyceraldehyde phosphodehydrogenase, the promoter of the gene for phosphoglycerate kinase and the endogenous promoter of cytochrome b5.

5. Strain according to Claim 3, **characterized in that** the inducible promoter is selected from the GAL10 and CYC1-GAL10 promoters.

6. Strain according to one of Claims 1 to 5, **characterized in that** the nucleic acid encoding human NADPH-cytochrome P450 reductase is integrated into the genome of the yeast.

7. Strain according to one of Claims 1 to 6, **characterized in that** the nucleic acid encoding human cytochrome b5 is integrated into the genome of the yeast.

8. Strain according to one of Claims 1 to 7, **characterized in that** it additionally comprises at least one nucleic acid encoding a human cytochrome P450.

9. Strain according to Claim 8, **characterized in that** the nucleic acid encoding the human cytochrome P450 is integrated on a plasmid.

10. Strain according to Claim 9, **characterized in that** it furthermore comprises an additional copy of the nucleic acid encoding human cytochrome b5 either on the said plasmid or integrated into the genome.

11. Strain according to one of the preceding claims, **characterized in that** it is haploid.

12. Strain according to any one of Claims 1 to 11, **characterized in that** the yeast is a strain of *Saccharomyces cerevisiae.*

13. Strain according to any one of Claims 1 to 7, 11 and 12, **characterized in that** it is a strain which comprises a nucleic acid encoding human cytochrome b5 and a nucleic acid encoding human NADPH-cytochrome P450 reductase.

14. Strain according to Claim 13, **characterized in that** the nucleic acid encoding human NADPH-cytochrome P450 reductase in this strain is under the control of the promoter of the gene for yeast glyceraldehyde phosphodehydrogenase.

15. Strain according to Claim 13, **characterized in that** the nucleic acid encoding human cytochrome b5 in this strain is under the control of the promoter of the gene for yeast phosphoglycerate kinase.

16. Strain according to one of Claims 13, 14 or 15, **characterized in that** the nucleic acid encoding human cytochrome b5 in this strain is under the control of the promoter of the gene for yeast phosphoglycerate kinase and the nucleic acid encoding human NADPH-cytochrome P450 reductase in this strain is under the control of the promoter of the gene for yeast glyceraldehyde phosphodehydrogenase.

17. Strain according to Claims 13 to 16, **characterized in that** it furthermore comprises at least one nucleic acid encoding a human cytochrome P450.

18. Strain according to Claim 17, **characterized in that** the nucleic acid encoding the human cytochrome P450 is integrated on a plasmid.

19. Genetically modified yeast strain which comprises a nucleic acid encoding human NADPH-cytochrome P450 reductase and whose genes encoding yeast cytochrome b5 and yeast NADPH-cytochrome P450 reductase have been inactivated.

20. Strain according to Claim 19, **characterized in that** the nucleic acid encoding human NADPH-cytochrome P450 reductase is under the control of the promoter of the yeast glyceraldehyde phosphodehydrogenase gene.

21. Process for obtaining strains according to Claim 13 or Claim 15, **characterized in that** it employs a yeast strain which comprises a nucleic acid encoding human NADPH-cytochrome P450 reductase under the control of the GAL10-CYC1 promoter and whose genes encoding yeast cytochrome b5 and yeast NADPH-cytochrome P450 reductase have been inactivated.

22. Process for obtaining strains according to Claim 14 or 16, **characterized in that** it employs a yeast strain which comprises a nucleic acid encoding human NAPDH-cytochrome P450 reductase under the control of the glyceraldehyde phosphodehydrogenase promoter and whose genes encoding yeast cytochrome b5 and yeast NADPH-cytochrome p450 reductase have been inactivated.

23. Process for evaluating the toxicity of a compound, **characterized in that**:
- the said compound is brought into contact with a yeast according to one of claims 1 to 18 or with an enzyme preparation which is derived from such a yeast, and
- the toxicity of the metabolites which are produced is analysed.

24. Method for determining in vitro the human metabolites of a chemical compound, **characterized in that**:
- the said compound is brought into contact with a yeast according to one of Claims 1 to 18 or with an enzyme preparation which is derived from such a yeast, and
- the metabolites which are produced are analysed.

25. Plasmid, **characterized in that** it enables a cytochrome P450, which is preferably human, and human microsomal cytochrome b5 to be expressed simultaneously in the yeast.

26. Plasmid according to Claim 25, **characterized in that**:
(i) the cassette for expressing the P450 preferably includes the GAL10-CYC1 promoter,
(ii) the cassette for expressing the b5 preferably includes either the GAL10-CYC1 promoter or the PGK promoter or the GAPDH promoter, and
(iii) when the same promoter is used twice, the two copies are present in opposite orientations on the plasmid.

27. Plasmid according to Claim 26, **characterized in that** the two expression cassettes are separated on the plasmid by, on the one hand, the yeast origin of replication and, on the other hand, at least one yeast selection marker.

28. Yeast strain which is transformed with a plasmid according to one of Claims 25 to 27.

29. Yeast strain according to Claim 28 which lacks the endogenous gene for microsomal cytochrome b5.
